(19) Europäisches Patentamt
European Patent Office
Office européen des brevets

(11) Numéro de publication : **0 446 097 A1**

(12) **DEMANDE DE BREVET EUROPEEN**

(21) Numéro de dépôt : **91400496.5**

(22) Date de dépôt : **25.02.91**

(51) Int. Cl.$^5$ : **C07D 333/20**, C07D 307/52, C07D 307/54, C07D 333/24, C07D 213/38, C07D 213/57, C07C 211/27, C07C 217/74, C07C 255/43, C07D 409/06, A61K 31/38

(30) Priorité : **26.02.90 US 484493**
**28.02.90 FR 9002493**

(43) Date de publication de la demande :
**11.09.91 Bulletin 91/37**

(84) Etats contractants désignés :
**AT BE CH DE DK ES FR GB GR IT LI LU NL SE**

(71) Demandeur : **JOUVEINAL S.A.**
**Tour Maine Montparnasse 33 Avenue du Maine**
**F-75755 Paris Cedex 15 (FR)**

(72) Inventeur : **Aubard, Gilbert**
**7, chemin de la Savetière**
**F-91129 Palaiseau (FR)**
Inventeur : **Calvet, Alain**
**Résidence Ronsard, 3, rue de Gatine**
**F-94240 L'Hay les Roses (FR)**
Inventeur : **Gouret, Claude-Jean**
**34, rue Croix du Val**
**F-92190 Meudon (FR)**
Inventeur : **Grouhel, Agnès**
**2, rue des Peupliers**
**F-92190 Meudon (FR)**
Inventeur : **Hudspeth, James**
**3839 Northland St.**
**Newbury Park, California 91320 (US)**
Inventeur : **Jacobelli, Henri**
**65, Av. du Général de Gaulle**
**F-91550 Paray Vieille Poste (FR)**
Inventeur : **Junien, Jean-Louis**
**36, Avenue Eiffel**
**F-92310 Sevres (FR)**
Inventeur : **Pascaud, Xavier**
**41, rue de Charenton**
**F-75012 Paris (FR)**
Inventeur : **Roman, François**
**11, allée Pierre Fresnay**
**F-94400 Vitry Sur Seine (FR)**
Inventeur : **Soulard, Claude**
**146, rue des Sources**
**F-92160 Antony (FR)**
Inventeur : **Yuan, Lin**
**426 N. Elisabeth Avenue AB, Bat, B.**
**Monterey Park, California 91754 (US)**

(54) **Ethylamines substituées, leur procédé de préparation, leur utilisation comme médicament et leurs intermédiaires de synthèse.**

(57) Ethylamines de formule générale (I)

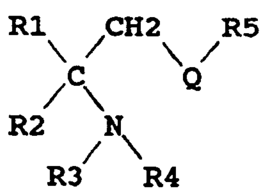

(I)

dans laquelle :
R1 est un radical hétéroaryle ou est phényle éventuellement mono, di ou tri-substitué,
R2 est alkyle,
R3 et R4 sont l'hydrogène ou des radicaux alkyle inférieur sans toutefois représenter tous deux l'hydrogène,
R5 est un hétéroaryle ou est un radical phényle éventuellement mono, di ou tri-substitué,
Q est éthylène-1,2-diyle ou un groupe cyclopropane-1,2-diyle, et leurs sels d'addition avec des acides.
Médicament comprenant une éthylamine (I), destiné notamment au traitement des affections neurologiques et/ou psychiques.

EP 0 446 097 A1

# ETHYLAMINES SUBSTITUEES, LEUR PROCEDE DE PREPARATION, LEUR UTILISATION COMME MEDICAMENT ET LEURS INTERMEDIAIRES DE SYNTHESE

La présente invention concerne de nouvelles éthylamines substituées, leur procédé de préparation, leur utilisation comme médicaments, et leurs intermédiaires de synthèse.

Ces éthylamines répondent à la formule générale (I)

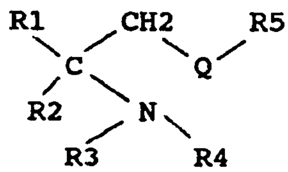

(I)

dans laquelle :

– Q représente un groupe éthylène-1,2-diyle (-CH=CH-) ou un groupe cyclopropane-1,2-diyle

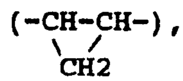

– R1 est un hétérocycle aromatique de 5 à 7 chainons dans lequel l'unique hétéroatome est l'azote, l'oxygène ou le soufre,

ou, sous réserve que Q représente un groupe cyclopropane-1,2-diyle ou sous réserve que R5 représente un radical hétérocyclique aromatique tel qu'il est défini ci- dessous, R1 est un radical phényle éventuellement mono, di ou tri substitué par des radicaux identiques ou différents qui sont alkyle ou alkoxy inférieurs,

– R2 est alkyle inférieur,

– R3 et R4, identiques ou différents, sont l'hydrogène ou des radicaux alkyle inférieurs sans toutefois représenter tous deux l'hydrogène,

– R5 est un hétérocycle aromatique de 5 à 7 chainons dans lequel l'unique hétéroatome est l'azote, l'oxygène ou le soufre,

ou est un radical phényle éventuellement mono, di ou tri-substitué par des radicaux identiques ou différents qui sont alkyle ou alkoxy inférieur,

Dans les définitions précédentes, le qualificatif "inférieur" comprend les radicaux ayant de 1 à 5 atomes de carbone en chaîne linéaire ou ramifiée.

On préfère les composés dans lesquels :

– R1 est un hétérocycle aromatique de 5 chainons dans lequel le seul hétéroatome est l'oxygène comme dans un radical 2-furyle ou 3-furyle ou bien est le soufre dans un radical 2-thiènyle ou 3-thiènyle ou encore est l'azote dans un radical 2-pyridyle, 3-pyridyle ou encore 4-pyridyle,

ou est un radical phényle pouvant être mono, di ou tri substitué par des radicaux alkoxy inférieurs comme méthoxy, sous réserve que Q représente un groupe cyclopropane-1,2- diyle ou sous réserve que R5 soit un hétérocycle aromatique tel que décrit ci-dessus,

– R2 est un radical méthyle, éthyle, n-propyle ou isopropyle,

– R3 et R4 sont l'hydrogène ou alkyle inférieur comme méthyle, sans toutefois être tous deux l'hydrogène,

– R5 est un hétérocycle aromatique de 5 chainons dans lequel le seul hétéroatome est l'oxygène comme dans un radical 2-furyle ou 3-furyle ou encore le soufre dans un radical 2-thiènyle ou 3-thiènyle,

ou encore est phényle éventuellement mono, di ou tri substitué par des radicaux alkoxy inférieurs comme méthoxy.

L'invention comprend également les sels d'addition des éthylamines de formule (I) ainsi que ceux de leurs isomères optiques ou géométriques qui font partie intégrante de cette invention, avec les acides minéraux ou organiques. Parmi ces sels, ceux obtenus par réaction des éthylamines avec des acides pharmaceutiquement acceptables sont préférés. Par exemple, ce sont ceux préparés avec les acides acétique, benzènesulfonique, camphosulfonique, citrique, éthanesulfonique, fumarique, bromhydrique, chlorhydrique, lactique, maléique, malique, méthanesulfonique, mucique,nitrique, pamoïque,phosphorique, salicylique, stéarique,succinique, sulfurique, tartrique.

Les éthylamines (I) de l'invention sont peu toxiques chez l'animal et présentent d'intéressantes propriétés

psychotropes qui justifient leur utilité en thérapeutique sous forme de médicaments.

Pour cette application, les composés préférés sont ceux dans lesquels R1 est un radical furyle ou thiènyle, R5 est phényle, furyle ou thiènyle, R2 est éthyle, R3 et R4 identiques sont méthyle. Plus particulièrement ce sont les éthylamines (I) suivantes :

– le 4-N,N-diméthylamino-1-phényl-4-(3-thiényl)-hex-1-ène et ses sels,
– Le trans 1-[2-N,N-diméthylamino-2-(3-thiényl)butyl]-2-phényl-cyclopropane et ses sels,
– le 4-N,N-diméthylamino-4-(2-furyl)-1-phényl-hex-1-ène et ses sels,
– le trans 1-[2-N,N-diméthylamino-2-(2-furyl)butyl]-2-phényl-cyclopropane et ses sels.

L'invention vise également un procédé de préparation des éthylamines de formule (I), caractérisé en ce qu'il consiste essentiellement comme montré au schéma 1,

- pour obtenir une éthylamine de formule (I) dans laquelle R3 est méthyle et R4 est l'hydrogène,

i) à réduire un isocyanate de formule (II) ou un isonitrile de formule (II'),

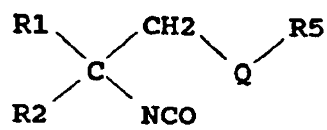
(II)

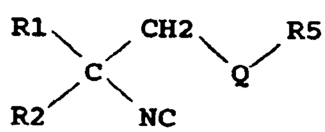
(II')

par un hydrure métallique ou organo métallique (Hm) de formule générale

M1(t) M2 H(r) Rx(s) (Hm)

dans lequel :

M1 représente un métal alcalin qui est de préférence le lithium ou le sodium et dont l'indice (t) a pour valeur 0 ou 1,

M2 est un métal du groupe III de la classification périodique des éléments et de préférence le bore ou l'aluminium,

(r) est l'indice représentatif du nombre d'atomes d'hydrogène de l'hydrure et a pour valeurs 1, 2, 3 ou 4,

Rx représente un groupe carbonitrile, un radical alkyle ou alkoxy inférieur dont l'indice (s) a pour valeurs 0, 1, 2 ou 3,

les indices (t), (r) et (s) ci dessus précisés répondant à la relation : (r) + (s) - (t) = 3

Les hydrures (Hm.1) dans lesquels M2 est l'aluminium ou est le bore lorsque (r) = 3, (t) et (s) ayant alors pour valeur 0, sont ceux préférés pour la réduction des composés (II) et (II')

ii) ou bien à hydrolyser un isocyanate de formule (II) ou un isonitrile de formule (II') pour obtenir une amine (III) de formule

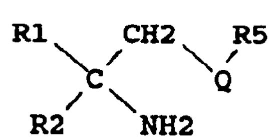
(III)

SCHEMA 1

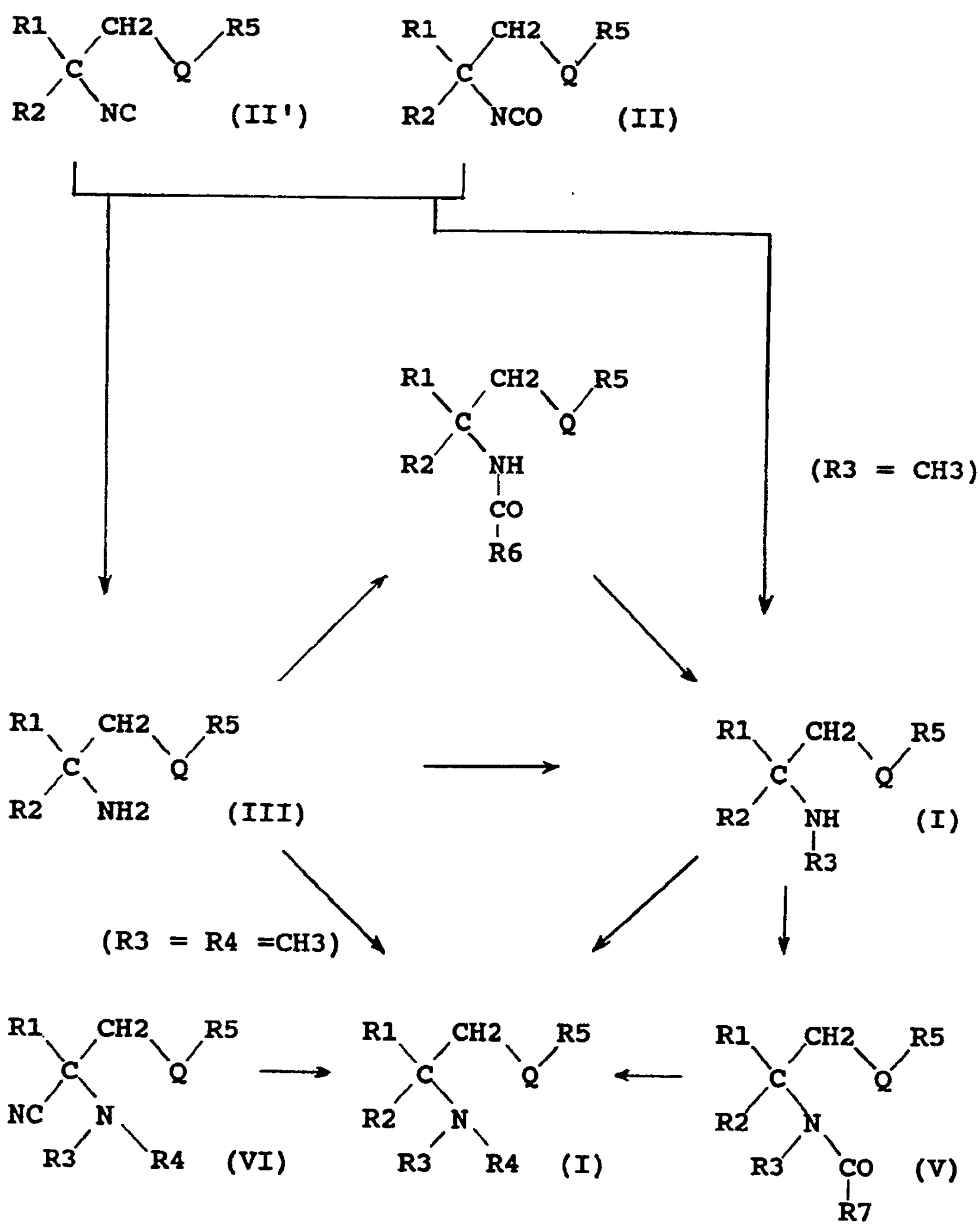


que l'on acyle par l'acide formique en présence de N,N'-carbonyldiimidazole en un composé intermédiaire N-formyl :

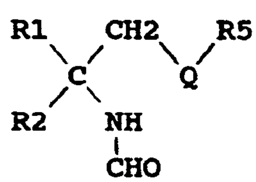

que l'on réduit par un hydrure (Hm.2) de formule (Hm) dans laquelle M2 est l'aluminium ou lorsqu'il représente le bore (r) a pour valeur 3 et (t) a pour valeur 0,

- et, pour obtenir une éthylamine de formule (I) dans laquelle R3 est alkyle inférieur différent de méthyle et R4 est l'hydrogène,

i) à alkyler une amine (III) par un halogénure Z1R3 dans lequel R3 a les significations précédemment énoncées et Z1 est le chlore, le brome ou l'iode, ou

ii) à acyler une amine (III) par un réactif (R6-CO)nZ2 dans lequel R6 est l'homologue carboné directement inférieur à R3 (R3=-CH2-R6) et n a pour valeur 1 lorsque Z2 est un halogène comme le chlore ou le brome ou est un radical hydroxyle, et n a pour valeur 2 lorsque Z2 représente un atome d'oxygène pour obtenir un carboxamide intermédiaire (IV) de formule

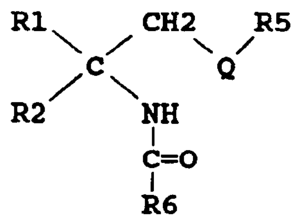

(IV)

puis à le réduire par un hydrure (Hm.2) précédemment défini,

- pour obtenir une éthylamine de formule (I) dans laquelle R3 et R4 identiques sont méthyle, à diméthyler une amine (III) en la faisant réagir avec le formaldéhyde et avec soit l'acide formique soit un hydrure réducteur métallique ou organo-métallique (Hm.3) de formule (Hm) dans laquelle M2 est le bore, et parmi lesquels les préférés sont ceux où Rx représente un groupe carbonitrile,

- pour obtenir une éthylamine (I) dans laquelle R3 et R4 sont alkyle inférieur et sont différents,

i) à acyler une éthylamine (I) dans laquelle R3 est alkyle inférieur et R4 est l'hydrogène par un agent R7-COZ5 dans laquelle R7 est l'homologue immédiatement inférieur de R4 (R4 = -CH2-R7) et Z5 représente le brome ou le chlore, pour obtenir le carboxamide (V)

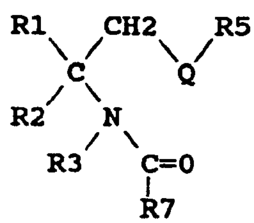

(V)

puis à le réduire par un hydrure (Hm.2) précédemment défini,

ii) ou à faire réagir un réactif organomagnésien R2MgZ3 dans lequel R2 est alkyle inférieur et Z3 un atome de chlore, de brome, ou d'iode sur un amino-nitrile (VI)

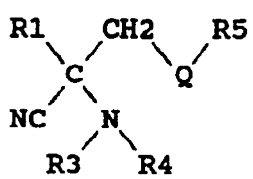

(VI)

dans lequel R3 et R4 identiques ou différents sont alkyle inférieur,et

- pour obtenir une éthylamine (I) dans laquelle R3 est alkyle inférieur et R4 est méthyle, à N-méthyler une éthylamine (I) dans laquelle R3 est alkyle inférieur et R4 est l'hydrogène par l'aldéhyde formique et un réducteur tel qu'un hydrure métallique ou organométallique (Hm.3) précédemment défini.

L'invention vise également les intermédiaires (XX) de formule générale

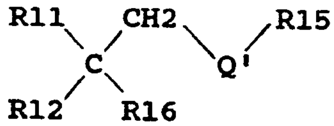

(XX)

dans laquelle :

– Q' représente un groupe éthylène-1,2-diyle (-CH=CH-) ou un groupe cyclopropane-1,2-diyle

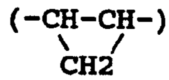,

– R11 est un hétérocycle aromatique de 5 à 7 chainons dans lequel l'unique hétéroatome est l'azote, l'oxygène ou le soufre,

ou, sous réserve que Q' représente un groupe cyclopropane-1,2-diyle ou sous réserve que R15 représente un hétérocycle aromatique tels qu'ils sont définis ci-dessous, R11 est un radical phényle éventuellement mono, di ou tri substitué par des radicaux identiques ou différents qui sont alkyle ou alkoxy inférieurs,

– R12 est alkyle inférieur ou un radical carbonitrile -CN,

– R15 est un hétérocycle aromatique de 5 à 7 chainons dans lequel l'unique hétéroatome est l'azote, l'oxygène ou le soufre,

ou est un radical phényle éventuellement mono, di ou tri-substitué, par des radicaux identiques ou différents qui sont alkyle ou alkoxy inférieur,

– R16 représente un radical isocyanate -NCO ou un radical isonitrile -NC, ou encore un radical -N(R13)R14 dans lesquels R13 et R14 sont l'hydrogène, alkyle inférieur ou encore des radicaux R6-CO- ou R7-CO-dans lesquels R6 et R7 sont l'hydrogène ou alkyle qui sont les homologues carbonés inférieurs à R13 ou R14 (R13 = -CH2-R6; R14 = -CH2-R7).

SCHEMA 2

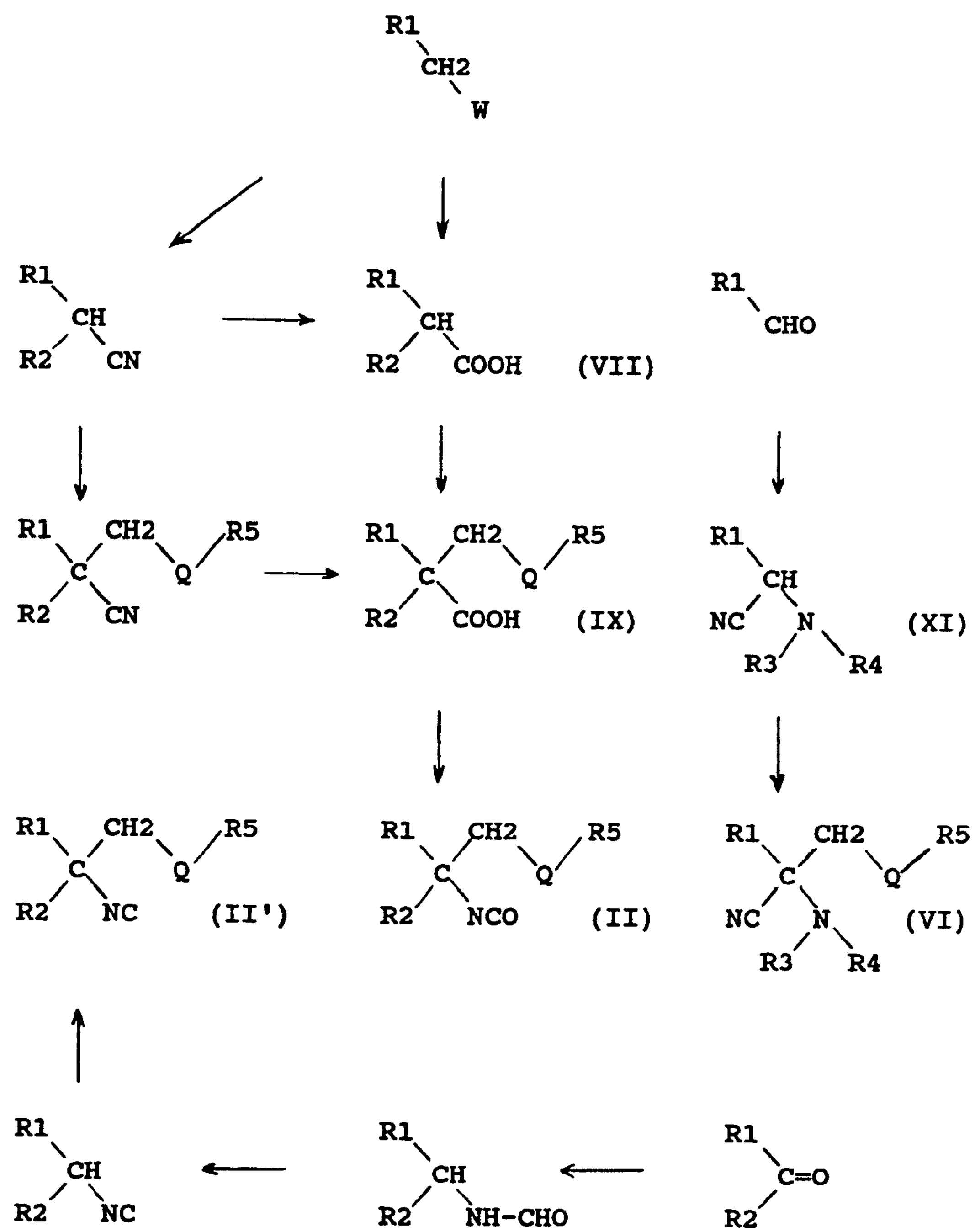

Le procédé de préparation des composés intermédiaires (XX) de formules (II), (II'), (III), (IV), (V) et (VI) consiste comme il est montré aux schémas 1 et 2 :

– pour préparer les composés isocyanates(II), à alkyler un composé R1-CH2-W dans lequel W est un radical carbonitrile (-CN) ou carboxyle (-COOH) par un halogènure d'alkyle de formule R2Z6, Z6 étant un halogène, pour obtenir,

pour W = -COOH un acide de formule (VII) R1(R2)-CH-COOH,

et, pour W = -CN, un nitrile de formule R1(R2)-CH-CN qui est hydrolysé en acide (VII),

puis à alkyler l'acide (VII) par un réactif (VIII) de formule Z7-CH2-Q-R5, Z7 étant un halogène ou un radical alkylsulfonyloxy en un acide (IX) R1(R2)C(COOH)CH2-Q-R5 et ensuite à préparer les isocyanates (II) par la réaction de Curtius, et,

– pour préparer les composés isonitriles (II') a utiliser, par exemple, un procédé adapté de celui décrit à la demande européenne de brevet n° 0 298 703 et qui consiste à faire réagir une cétone R1-CO-R2 avec le formamide et l'acide formique pour préparer le formamide N-substitué de formule R1(R2)CH-NH-CHO que l'on déshydrate par l'oxychlorure de phosphore pour en obtenir l'isonitrile correspondant R1(R2)CH-NC qui, alkylé par le réactif (VIII) déja décrit, conduit à l'isonitrile (II'), et,

– pour préparer une amine (III), à hydrolyser soit un isocyanate (II) soit un isonitrile (II'), et,

– pour préparer un carboxamide intermédiaire (IV), à acyler une amine (III) par un réactif (R6-CO)nZ2 déjà défini, et,

– pour préparer un carboxamide intermédiaire (V), à acyler une éthylamine (I)

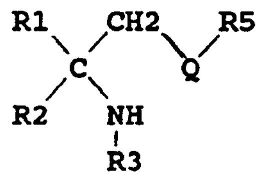

par un halogènure de formule R7-COZ5, dans laquelle R7 est alkyle inférieur et Z5 est le brome ou le chlore et,

– pour préparer un amino nitrile (VI) à faire réagir un aldéhyde R1-CHO avec une amine de formule R3-NH-R4 et un cyanure de métal alcalin pour obtenir l'aminonitrile intermédiaire (XI) R1(CN)CH-N(R3)R4 dans lequel R3 et R4 sont tous deux alkyle inférieur, puis à l'alkyler par un réactif (VIII) déjà décrit.

Les composés (I) de l'invention se différencient des composés connus les plus proches de par leur structure chimique et également de par leur application.

Ainsi, à la demande de brevet européen n° 0 298 703 il est décrit des dérivés du thiophène de formule :

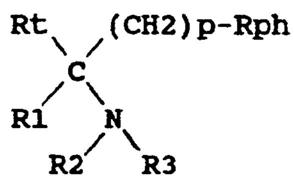

dans laquelle, au sens le plus large,

– Rt est un radical thiènyle,
– R1, R2 et R3 représentent des radicaux alkyle inférieur,
– Rph est un radical phényle éventuellement substitué,
– p a pour valeurs 1, 2 ou 3,

et, dans laquelle, pour les composés préférés,

– R1 est un radical éthyle,
– R2 et R3 sont des radicaux méthyle,
– Rph est un radical phényle, 3,4-diméthoxyphényle, 3,4,5-triméthoxyphényle ou 4-chlorophényle,
– p a pour valeurs 1 ou 3.

Sans démonstration à l'appui, les composés sont présentés comme peu toxiques et comme ayant un effet régulateur de la motilité du tractus gastro intestinal caractérisé par un effet stimulant sur un tractus d'activité ralentie et, inversement, par un effet inhibiteur sur un tractus hyperactif.

Différents de par leur structure chimique, notamment par la nature de la chaine carbonée reliant les deux sites aromatiques, les composés de la demande européenne n° 0 298 703 diffèrent aussi des éthylamines (I) de l'invention par leurs propriétés. Essentiellement il n'est pas rapporté d'activité de type psychotrope pour les composés de la demande EP 0 298 703, alors que pour les éthylamines (I), leur activité biologique permet d'envisager leur application au traitement des affections neuro-psychiques.

Parmi les procédés de préparation des composés (I) qui ont été précédemment décrits et présentés au schéma 1, on préfère, lorsque R3 et R4 sont alkyle inférieur, utiliser la méthode qui consiste à faire réagir un intermédiaire amino nitrile (VI) avec un dérivé organo magnésien R2MgZ3.

D'une façon explicite ce procédé consiste :

i) à faire réagir un aldéhyde de formule R1-CHO avec une amine secondaire R3-NH-R4 pour obtenir l'amino

nitrile (XI) de formule NC(R1)-CH-N(R3)(R4).

Cette réaction est fréquemment utilisée pour la préparation d'amino-acides selon la méthode de Strecker. Elle est appliquée à la synthèse des composés (XI) et consiste à faire réagir 1 mole d'aldéhyde avec 0,8 à 3,0 mole de cyanure de sodium ou de potassium et avec 0,8 à 3,0 mole d'un sel d'amine secondaire de formule R3-NH-R4 en milieu alcoolique ou hydro-alcoolique, à une température comprise entre 5°C et la température de reflux du milieu et pendant de 1 à 24 h.

Les sels d'amines secondaires utilisés de préférence sont hydrosolubles comme, entre autres, les chlorhydrate, bromhydrate, sulfate.

Le solvant réactionnel comprend un alcool de bas poids moléculaire et miscible en toutes proportions avec l'eau comme le méthanol ou l'éthanol. Dans le cas de milieux hydro-alcooliques les proportions respectives sont comprises entre 95 et 10% d'alcool pour leur complément à 100% par l'eau, ce rapport permettant d'obtenir, de façon favorable, un milieu réactionnel homogène.

D'une manière courante à une solution de 1,1 à 1,3 mole de cyanure de sodium et de 1,1 à 1,3 mole de chlorhydrate d'amine secondaire en solution dans 150 à 400 ml d'eau il est ajouté 1,0 mole d'aldéhyde en solution dans 75 à 200 ml de méthanol. Le mélange est agité durant 3 à 5 h à une température comprise entre 15 et 30°C puis traité pour isoler l'amino-nitrile (XI) qui est, si nécessaire, purifié par distillation.

ii) puis à alkyler ce composé avec un réactif (VIII) pour obtenir l'intermédiaire de l'invention (VI).

La réaction consiste dans un premier temps à préparer l'anion de l'amino nitrile par action d'une base forte. A cet effet on préfère le N,N-diisopropyl amidure de lithium (LDA) que l'on prépare "in situ" à partir de quantités équimoléculaires de diisopropylamine et de butyllithium. Pour une mole de LDA ainsi préparé on ajoute ensuite dans le THF de 1,0 à 0,6 mole d'intermédiaire (XI) pour en obtenir l'anion. Le réactif (VIII) est ensuite introduit à une température comprise entre -10 et 50°C puis le mélange laissé à réagir de 2 à 48 h selon la réactivité des composés.

Ainsi, d'une manière préférée, à une mole de diisopropylamine dans 500 ml de THF il est ajouté à -20°C environ de 0,95 à 1 mole de butyllithium puis de 0,8 à 1,0 mole d'intermédiaire (XI) en solution dans environ 500 ml de THF. Après réaction de 1 à 2 heures entre 20 et 100°C pour former l'anion, le mélange est refroidi vers 0°C et on y ajoute de 0,8 à 1,0 mole du réactif (VIII).

La réaction est maintenue durant 1 à 2 h à température ambiante puis le mélange est traité pour isoler et purifier l'amino nitrile intermédiaire (VI) obtenu,

iii) puis à faire réagir sur l'intermédiaire (VI) un réactif organométallique comme un dérivé organo magnésien de Grignard de formule R2MgZ3 dans lequel Z3 est un halogène et plus particulièrement le brome ou le chlore, selon une réaction décrite comme par exemple par N.J. Léonard et coll., J. Am. Chem. Soc., 1956, 78, p. 1986 et 1957, 79, p. 5279. Cette substitution du radical nitrile du composé (VI) par le radical R2 alkyle du dérivé organo magnésien est effectuée dans les éthers comme l'éther diéthylique, le méthyl-t.butyl éther, les éthers di-isopropylique ou dibutylique ou encore le tétrahydrofurane qui est le solvant préféré, et consiste pour une mole de composé (VI) à faire réagir 1,5 à 6 mole de dérivé organo magnésien à une température comprise entre 5 et 50°C et ce durant 30 minutes à 12 heures.

De préférence la méthode consiste à ajouter à une température comprise entre 10 et 20°C une mole de composé (VI), éventuellement en solution dans le THF, à 4 à 5 mole du composé organomagnésien également en solution dans le THF. La réaction est poursuivie durant de 2 à 5 heures à la même température puis le complexe obtenu décomposé par addition de solution aqueuse de chlorure d'ammonium. Après traitements, l'éthylamine (I) est isolée et purifiée.

Les modes opératoires qui suivent illustrent, sans pour autant la limiter, la préparation de dérivés intermédiaires essentiels et celle des éthylamines (I), de l'invention.

Les produits sont, selon les réactions effectuées, obtenus tels quels dans un état de pureté satisfaisant, ou purifiés par des techniques appropriées indiquées dans les exemples et qui sont généralement la cristallisation, la distillation sous vide ou encore la chromatographie sur colonne. Dans ce dernier cas on utilise favorablement la technique dite de "chromato-flash" sur un support de silice (marque "Merck", produit Kieselgel 60, granulométrie 230 à 400 mesh).

Par ailleurs, la pureté, l'identité et les caractéristiques physico chimiques des produits préparés sont rapportées et déterminées par :

– leur point d'ébullition sous la valeur du vide lors de leur distillation (en pascal),

– leur point de fusion, déterminé par la méthode du tube capillaire et dont la valeur indiquée n'est pas corrigée,

– la chromatographie sur couches minces (CCM) de silice (plaques prêtes à l'emploi - produit "Merck" réf. 60 F 254) - selon une technique qui est brièvement rappelée : les produits à étudier sont déposés sur la plaque à raison de 100 mcg environ puis élués de façon ascendante par des solvants ou leurs mélanges qui sont énumérés ci-après, les proportions respectives étant indiquées en volumes pour volumes,

| | | | |
|---|---|---|---|
| réf. S.A - | hexanes 100 / | acétate d'éthyle | 10 |
| S.B - | " 60 / | " | 10 |
| S.C - | " 40 / | " | 10 |
| S.D - | " 20 / | " | 10 |
| S.E - | " 10 / | " | 10 |
| S.F - | dichlorométhane 20 / | hexanes | 80 |
| S.G - | dichlorométhane | | |
| S.H - | " 90 / | acétone | 10 |
| S.I - | " 85 / | " | 15 |
| S.J - | " 80 / | " | 20 |
| S.K - | " 98 / | méthanol | 2 |
| S.L - | " 95 / | " | 5 |
| S.M - | " 90 / | " | 10 |
| S.N - | " 85 / | " | 15 |

Après développement, les chromatogrammes sont observés sous lumière ultra violette de 254 nm de longueur d'onde et/ou après révélation colorée par pulvérisation du réactif de Dragendorff ou du réactif à la tolidine. Les Rf observés ainsi que les références des solvants d'élution utilisés sont indiqués dans les exemples.

- l'analyse centésimale élémentaire dont les résultats, conformes aux normes admises ne sont pas reportés de façon chiffrée mais sont signalés être effectués par la représentation de l'élément dosé,
- la spectrographie infra rouge des composés en pastilles dans le KBr ou sous forme de films entre deux fenêtres de NaCl ou encore en suspension dans le nujol (R) ou encore en solution dans le CCl4 ; les absorbtions les plus intenses sont rapportées par la valeur de leur longueur d'onde en cm-1
- la résonance magnétique nucléaire du proton (RMN) est étudiée à 60 ou 90 MHz, les produits étant solubilisés dans le deutérochloroforme. L'aspect des signaux, leur déplacement chimique exprimé en p.p.m par rapport au tétraméthylsilane utilisé comme référence interne sont indiqués. Les protons dits "échangeables" après addition d'oxyde de deutérium sont également signalés.

PREPARATION DES INTERMEDIAIRES

- Composés de formule (XI)

XI.1/ α-diméthylamino-phénylacétonitrile

(R1 = C6H5 ; R3 = R4 = CH3)

Dans un réacteur, à une solution de 11,82 g (0,241 mol) de cyanure de sodium et de 19,61 g (0,240 mol) de chlorhydrate de diméthylamine dans 40 ml d'eau, il est ajouté en une heure, à une température comprise entre 30 et 40°C une solution de benzaldéhyde (0,200 mol) dans 20 ml de méthanol. Le mélange est agité 4 h à la température ambiante, puis précipité dans 150 ml d'eau glacée et extrait à l'éther.

Les phases éthérées sont lavées successivement à l'eau, par une solution à 25 % de bisulfite de sodium, puis à nouveau à l'eau. Après évaporation de l'éther, le résidu est purifié par distillation. Eb/93 Pa = 74-79°C.

Poids = 30,4 g      Rdt = 95%

Selon ce mode opératoire et à partir des aldéhydes et des sels d'amines secondaires appropriés les α-amino-acétonitriles intermédiaires XI.2 à XI.6 ont été préparés.

XI.2 / α-diméthylamino-α-(3,4,5-triméthoxyphényl)-acétonitrile

(R1 = 3,4,5(CH30)3-C6H2 ; R3 = R4 = CH3)

Rdt = 54 %      F = 77°C (éth. pétr.)

XI.3 / α-diméthylamino-α-(2-pyridyl)-acétonitrile

(R1 = 2-pyridyle ; R3 = R4 = CH3)
Rdt = 67 % Eb/66 Pa = 98-110°C

XI.4 / α-diméthylamino-α-(2-furyl)-acétonitrile

(R1 = 2-furyle ; R3 = R4 = CH3)
Rdt = 58 % Eb/73 Pa = 55-75°C

XI.5 / α-diméthylamino-α-(2-thiènyl)-acétonitrile

(R1 = 2-thiènyle ; R3 = R4 = CH3)
Rdt = 84 % Eb/7 Pa = 75°-85°C

XI.6 / α-diméthylamino-α-(3-thiènyl)-acétonitrile

(R1 = 3-thiènyle ; R3 = R4 = CH3)
Rdt = 72 % Eb/200 Pa = 86°C

- Composés de formule (VIII).

VIII.1 / 1-(2-thiènyl)-3-chloroprop-1-ène

(R5 = 2-thiènyle ; Q = -CH=CH- ; Z7 = Cl)

– 1er stade : 104,06 g d'acide malonique (1,0 mol), 56,07 g (0,50 mol) de 2-thiophène carboxaldéhyde, 250 ml de pyridine et 5ml de pipéridine sont chauffés au bain marie 2 heures puis au reflux durant 5 minutes. Après refroidissement, la solution est précipitée dans l'eau et traitée par un excès d'acide chlorhydrique (250 ml de solution concentrée à 37 %) pour précipiter le produit qui est ensuite filtré puis recristallisé dans un mélange éthanol-eau pour obtenir l'acide 2-thiènylacrylique purifié.

Poids = 42,38 g Rdt = 58 % F = 143-144°C

– 2ème stade : 37,34 g (0,24 mol) de l'acide précédent, 30 ml (0,24 mol) de complexe BF3-éther dans 310 ml de méthanol sont chauffés au reflux durant 6 heures. La solution refroidie est précipitée dans l'eau puis extraite au dichlorométhane. Les phases organiques sont réunies, lavées par une solution saturée en NaHCO3 puis avec une solution saturée en NaCl puis déshydratées sur MgSO4. Le résidu solide de couleur brune qui est obtenu après élimination des solvants par distillation est recristallisé dans l'hexane pour obtenir le 2-thiènyl acrylate de méthyle purifié.

Poids = 32,65 g Rdt = 81 % F = 46-47°C

– 3ème stade : à un mélange de 5,28 g (39,6 mmol) de chlorure d'aluminium et de 40 ml d'éther diéthylique refroidi à -10°C et sous atmosphère d'azote on ajoute lentement sous agitation une suspension de 4,51 g (118,9 mmol) d'hydrure de lithium aluminium dans 150 ml de THF.

Une solution de 10,0 g (59,45 mmol) d'ester méthylique précédent dans 50 ml de THF est lentement ajoutée à -10°C puis la solution est agitée à la même température durant une heure et demie. Les complexes formés sont décomposés par addition d'une solution d'acide sulfurique 3M et le mélange est extrait à l'éther. Les phases éthérées réunies sont lavées par une solution saturée en NaHCO3 puis par une solution saturée en NaCl et déshydratées sur MgSO4. Par évaporation de l'éther sous vide on obtient 7,83 g (94 %) de produit résiduel sous forme d'une huile brune. Le 1-(2-thiènyl)-prop-1-èn-3-ol brut, instable à température ambiante, est conservé à une température inférieure à 0°C.

– 4ème stade : dans un mélange de 39,53 g (296 mmol) de N-chlorosuccinimide dans 180 ml de dichlorométhane anhydre à une température de 0°C on ajoute lentement 21,74 ml (296 mmol) de sulfure de diméthyle. Le mélange est refroidi à -10°C et une solution de 11,86 g (84,6 mmol) de l'alcool précédent dans 50 ml de dichlorométhane est ajoutée.

La température de la solution est ramenée à 0°C et y est maintenue 2 heures. Le mélange est dilué par 100 ml d'hexanes puis précipité dans 200 ml d'eau glacée. La phase organique est séparée et la phase aqueuse réextraite à l'éther. Les phases éthérées réunies sont lavées puis déshydratées. L'éther est éliminé par distillation sous vide, on obtient sous forme d'huile marron instable le 1-(2-thiènyl)-3-chloroprop-1-ène brut qui est utilisé tel quel.

Poids = 12,06 g Rdt = 94 %

VIII.2 / 1-(3-thièny)-3-chloro-prop-1-ène

(R5 = 3-thiènyle ; Q = -CH=CH- ; Z7 = Cl)

L'intermédiaire est obtenu à partir de 3-thiophène-carboxaldéhyde selon le procédé décrit à l'exemple précédent.

– 1er stade : acide 3-thiènylacrylique

F = 146°C (éthanol/eau)

– 2ème stade : 3-thiènylacrylate de méthyle

F = 49°C (hexanes)

– 3ème stade : 1-(3-thiènyl)-prop-1-èn-3-ol

huile non purifiée

– 4ème stade : 1-(3-thiènyl)-3-chloro-prop-1-ène

solide blanc amorphe non purifié

VIII.3 / 1-(2-furyl)-3-chloro-prop-1-ène

(R5 = 2-furyle ; Q = -CH=CH- ; Z7 = Cl)

L'intermédiaire est obtenu à partir de 2-furylcarboxaldéhyde en quatre stades selon le procédé décrit à la préparation de l'intermédiaire VIII.1

VIII.4 / trans-1-mésyloxyméthyl-2-phényl-cyclopropane

(R5 = C6H5 ; Z7 = CH3-SO3 ; Q = cyclopropane 1,2 diyle)

– 1er stade : on ajoute goutte à goutte sous atmosphère d'azote une solution de 25,0 g (154 mmol) d'acide trans-2-phényl-cyclopropanecarboxylique dans 100 ml de THF à une solution de complexe borane - THF. La solution est portée 3 heures au reflux puis on ajoute lentement 130 ml de solution NaOH 2N et le mélange est agité 30 minutes. Les extraits éthérés sont concentrés sous vide pour obtenir 20,78 g de trans-1-hydroxyméthyl-2-phényl-cyclopropane brut qui est purifié par distillation.

Poids = 18,19 g    Rdt = 80 %    Eb/33 Pa = 90-97°C

– 2ème stade : dans 100 ml de dichlorométhane on ajoute 9,18 g (66,2 mmol) de l'alcool obtenu ci-dessus et 13,83 ml (99,25 mmol) de triéthylamine. Sous atmosphère d'azote et à -10°C on ajoute goutte à goutte 5,63 ml (72,8 mmol) de chlorure de méthanesulfonyle. Le mélange est agité durant 15 minutes à -10°C puis lavé successivement à l'eau glacée, avec une solution à 10 % de HCl, une solution saturée en NaHCO3 puis avec une solution saturée en NaCl. Après déshydratation sur MgSO4 à 0°C la solution est concentrée sous vide pour obtenir une huile jaune. L'intermédiaire ainsi obtenu est dissout dans le THF anhydre et utilisé tel quel.

VIII.5 / trans-1-bromométhyl-2-phényl-cyclopropane

(R5 = C6H5 ; Z7 = Br ; Q = cyclopropane 1,2 diyle)

A 300 ml de dichlorométhane on ajoute 61,0 g (0,34 mol) de N-bromosuccinimide et l'on refroidit à 0°C sous atmosphère d'azote puis on ajoute goutte à goutte 29,4 ml (0,41 mol) de sulfure de diméthyle. Le mélange est agité à 0°C durant 30 minutes puis refroidi à -5°C ; on introduit alors goutte à goutte une solution de 33,6 g (0,23 mol) de trans-1-hydroxyméthyl-2-phényl-cyclo-propane obtenu à la préparation VIII.4 dans 100ml de chlorure de méthylène. Le mélange est agité durant 6 heures à 0°C puis 16 heures à 25°C ; on dilue alors par addition de 250 ml d'hexanes et le mélange est précipité dans 250 ml d'eau glacée. La phase organique est lavée par une solution saturée en NaCl puis déshydratée sur MgSO4. Les solvants sont éliminés par concentration sous vide et le résidu est purifié par distillation.

Poids = 40,81 g    Rdt = 85 %    Eb/27 Pa = 72°C

VIII.6/trans-1-mésyloxyméthyl-2-(3,4,5-triméthoxyphényl)-cyclopropane

(R5 = 3,4,5(CH30)3-C6H2 ; Z7 = CH3-SO3 ; Q = cyclopropane 1,2 diyle)

– 1er stade : dans 400 ml de méthanol on ajoute 55,0 g (0,23 mol) d'acide 3,4,5-triméthoxycinnamique et 28,39 ml (0,23 mol) de complexe BF3 - éther, la solution est chauffée 6 heures au reflux. Le mélange est refroidi puis précipité dans l'eau et extrait au dichlorométhane. Les phases organiques réunies sont traitées et desséchées puis le solvant éliminé par distillation sous vide. Le 3,4,5-triméthoxycinnamate de méthyle est purifié par recristallisation dans le méthanol.

Poids = 46,17 g Rdt = 80 % F = 96-98°C

– 2ème stade : à une solution de 20,15 ml (214 mmol) de 2-méthyl-2-propanol dans 125 ml de THF anhydre on ajoute lentement sous atmosphère d'azote, 106,83 ml (213,7 mmol) de n-butyllithium en solution 2M dans l'hexane. Après 30 minutes la solution est traitée en ajoutant goutte à goutte une solution dans 100 ml de THF de 17,97 g (71,22 mmol) d'ester méthylique précédent. Le milieu réactionnel est porté au reflux durant 2 heures et demie puis refroidie par un bain de glace et hydrolysée par addition de 200 ml d'eau. La phase aqueuse est extraite à l'acétate d'éthyle et les fractions organiques déshydratées sur MgSO4. Les solvants sont évaporés sous vide pour donner un résidu de 3,4,5-triméthoxycinnamate de t.butyle que l'on purifie par recristallisation de l'hexane.

Poids = 16,27 g Rdt = 78 % F = 83-85°C

– 3ème stade : à une suspension agitée de 15,47 g (70,3 mmol) d'iodure de triméthylsulfonium dans 150 ml de DMSO qui est maintenue à 25-30°C, on ajoute, sous azote, 3,0 g d'hydrure de sodium en dispersion huileuse à 60 % (75 mmol).

Après fin de dégagement d'hydrogène (30 minutes environ) on ajoute, sans dépasser 35°C une solution de 15,9 g (54 mmol) de l'ester t.butylique précédent dans 100ml de DMSO.

Le mélange est agité 30 minutes à 25-30°C puis une heure et demie à 55-60°C. Il est ensuite précipité dans 380 ml d'eau et extrait par l'acétate d'éthyle. Les extraits combinés sont déshydratés et concentrés sous vide pour obtenir le résidu de trans-2-(3,4,5-triméthoxyphényl)-1-cyclopropanecarboxylate de t.butyle sous forme d'une huile jaune qui se solidifie. Le produit est purifié par recristallisation de l'hexane.

Poids = 9,69 g Rdt = 58 % F = 68-70°C

– 4ème stade : 3,66 g (96,5 mmol) d'hydrure de lithium aluminium sont dispersés dans 150 ml de THF sous atmosphère d'azote. On ajoute lentement 9,92 g (32,2 mmol) de l'ester t.butylique précédent en solution dans 100 ml de THF. Le mélange est chauffé au reflux durant une heure et demie puis on additionne avec précautions 5,58 ml de solution NaOH 10% puis 7,32 ml d'eau. La suspension obtenue est maintenue sous agitation durant une nuit ; l'insoluble est filtré et le filtrat concentré sous vide pour obtenir le trans-1-hydroxyméthyl-2-(3,4,5-triméthoxyphényl)-cyclopropane brut que l'on purifie par distillation sous vide.

Poids = 6,65 g Rdt = 87 % Eb/3 Pa = 145-165°C

– 5ème stade : en pratiquant comme décrit au stade 2 de l'intermédiaire VIII.4 précédent avec le dérivé obtenu ci dessus on obtient le trans-1-mésyloxyméthyl-2-(3,4,5-triméthoxyphényl)-cyclopropane sous forme d'un solide blanc qui est utilisé sans autre purification.

VIII.7 / trans-1-chlorométhyl-2-(2-thiènyl)-cyclopropane

(R5 = 2-thiènyle ; Z7 = Cl ; Q = cyclopropane 1,2 diyle)

Selon les procédés des stades 2, 3 et 4 de l'exemple VIII.6 précédent on prépare le trans-1-hydroxyméthyl-2-(2-thiènyl)-cyclopropane à partir du 3-(2-thiényl)-propènoate de méthyle.

Dans 75,0ml de triéthylamine anhydre on ajoute 15,50g (100mmol) d'alcool et 40,0g de dichlorotriphénylphosphorane à 85% (102 mmol). Le mélange est agité sous atmosphère d'azote anhydre à 25°c durant 24 heures puis précipité dans 500 ml d'eau. On ajoute 100 ml d'hexanes, filtre et sépare la phase organique. La phase aqueuse est extraite à nouveau par 100 ml d'hexanes; les phases organiques réunies sont séchées sur Na2SO4 puis les solvants sont éliminés par distillation sous vide et sur bain marie. Le résidu huileux est purifié par distillation sous vide.

Poids = 9,7 g Rdt = 56 % Eb/40 Pa = 65-70°C

VIII.8 / trans-1-chlorométhyl-2-(3-thiènyl)-cyclopropane

(R5 = 3-thiènyle ; Z7 = Cl ; Q = cyclopropane 1,2 diyle)

Le composé est préparé à partir du 3-(3-thiényl) -propènoate de méthyle par un mode opératoire identique à celui décrit pour le composé VIII.7 précédent. Le produit est obtenu sous forme d'une huile jaune pâle qui est purifiée par distillation. Eb/400 Pa = 60-78°C

VIII.9 / trans-1-chlorométhyl-2-(2-furyl)-cyclopropane

(R5 = 2-furyle ; Z7 = Cl ; Q = cyclopropane 1,2 diyle)

Tel que décrit précédemment pour le composé VIII.7 le produit est préparé à partir du 3- (2-furyl)-propènoate de méthyle. La purification est réalisée par distillation sous vide. Eb/1,3 pa = 55-60°C

-α-amino acétonitriles de formule (VI)

Les composés sont préparés par alkylation des acétonitriles (XI) avec les réactifs (VIII) précédémment décrits.

Après réaction, les composés obtenus, souvent instables, sont soit purifiés par cristallisation soit engagés tels quels dans la suite des réactions.

La pureté et l'identité des produits est vérifiée par chromatographies sur couches minces et par RMN.

Mode opératoire général:

Dans un réacteur protégé de l'humidité et sous atmosphère d'azote, 1,025 mole de n. butyllithium (solution 10M/ hexanes) est ajoutée goutte à goutte à -20°C dans une solution de 1,025 mole de diisopropyl-amine dans 1 litre de tetrahydrofurane anhydre.

Le mélange est maintenu 15 minutes à - 20°C. A -72°C, il est introduit 1,0 mole de nitrile (XI) en solution dans 200 ml de THF, l'agitation est maintenue 1 h 30 à cette température puis on ajoute 1,025 mole de réactif (VIII) en solution dans 500 ml de THF. Après 20 minutes à -72°C le mélange est agité 1 h à température ambiante.

On ajoute ensuite 1,5 l de solution de NH4Cl à 10 % (p/v) et 750 ml de mélange hexanes-acétate d'éthyle 1-1 (v/v).

La phase organique est séparée, la phase aqueuse réextraite par le même mélange de solvants. Les phases organiques réunies sont lavées par extraction avec une solution saturée en chlorure de sodium, puis séchées sur MgSO4. Les solvants sont éliminés par distillation sous vide et sur bain-marie. Le résidu huileux est, selon les cas, cristallisé par addition d'hexanes, ou utilisé tel quel au stade suivant.

Selon ce mode opératoire les intermédiaires VI.1 à VI.22 sont préparés. Pour chacun de ces composés les acétonitriles (XI) et les réactifs (VIII) engagés sont indiqués.

VI.1/trans-1-(2-cyano-2-phényl-N,N-diméthylaminoéthyl)-2-phényl-cyclopropane.

(R1 = R5 = C6H5 ; R3 = R4 = CH3 ; Q = cyclopropane 1,2 diyle)
A partir de XI.1 et de VIII.4
Rdt = 57 % (brut) CCM : 0,65-0,70 ; S.C.
RMN : 0,60-0,90 (m,3H) ; 1,28 (m, 1H) ; 2,15 (m, 2H) ; 2,30 (d, 6H) ; 6,78-7,78 (m, 10H).

VI.2/trans-1-(2-cyano-2-(3,4,5-triméthoxyphényl)-N,N-diméthylaminoéthyl]-2-phényl-cyclopropane.

(R1 = 3,4,5(CH30)3-C6H2 ; R3 = R4 = CH3 ; R5 = C6H5 ; Q = cyclopropane 1,2 diyle)
A partir de XI.2 et de VIII.5
Rdt = 87 % (brut) CCM : 0,50-0,55 ; S.D.
RMN : 0,60-0,80 (m, 2H) ; 1,10-1,30 (m, 1H) ; 1,60-1,90 (m, 1H) ; 2,28 (d, 8H) ; 3,86 (m, 9H) ; 6,78 (d, 2H) ; 6,89-7,39 (m, 5H)

VI.3/ 1-cyano-1-N,N-diméthylamino-1-phényl-4-(2-thiényl) -but-3-ène.

(R1 = C6H5 ; R3 = R4 = CH3 ; R5 = 2-thiènyle ; Q = -CH=CH-)
A partir de XI.1 et de VIII.1
Rdt = 80 % (brut) CCM : 0,70 ; S.D.
RMN : 2,23 (s, 6H) ; 2,80 (d, 2H) ; 5,88-6,19 (dt, 1H) ; 6,60 (d, 1H) ; 6,87-7,38 (m, 8H)

VI.4/ 1-cyano-1-N,N-diméthylamino-1-phényl-4-(3-thiényl) -but-3-ène.

(R1 = C6H5 ; R3 = R4 = CH3 ; R5 = 3-thiènyle ; Q = -CH=CH-)
A partir de XI.1 et de VIII.2
Rdt = 75 % (brut) CCM : 0,60 ; S.A.
RMN : 2,30 (s, 6H) ; 2,85 (m, 2H) ; 5,40 (m, 1H) ; 6,30 (d, 1H) ; 6,90-7,80 (m, 8H)

VI.5/trans-1-(2-cyano-2-phényl-N,N-diméthylaminoéthyl) -2-(3,4,5-triméthoxyphényl)-cyclopropane.

(R1 = C6H5 ; R3 = R4 = CH3 ; R5 = 3,4,5 (CH30)3 -C6H2 ; Q = cyclopropane 1,2 diyle)

A partir de XI.1 et de VIII.6
Rdt = 78 % (brut) CCM : 0,35-0,40 ; S.D.
RMN : 0,55-1,45 (m, 4H) ; 2,20 (d, 2H) ; 2,35 (s, 6H) 3,85 (s, 9H) ; 6,28 (s, 2H) ; 7,50 (m, 5H)

VI.6/trans-1-[2-cyano-2-(2-pyridyl)-N,N-diméthylamino-éthyl]-2-phényl-cyclopropane.

(R1 = 2-pyridyl ; R3 = R4 = CH3 ; R5 = C6H5 ; Q = cyclopropane 1,2 diyle)
A partir de XI.3 et de VIII.5
Rdt = 79 % (brut) CCM : 0,25 ; S.E.
RMN : 0,62-1,65 (m, 4H) ; 2,35 (s, 6H) ; 2,40 (d, 2H) ; 7,16 (m, 6H) ; 7,65 (m, 1H) ; 7,75 (m, 1H) ; 8,78 (m, 1H)

VI.7/ 1-cyano-1-N,N-diméthylamino-1-(2-furyl)-4-phényl--but-3-ène.

(R1 = 2-furyle ; R3 = R4 = CH3 ; R5 = C6H5 ; Q = -CH=CH-)
A partir de XI.4 et de chlorure de cinnamyle
Rdt = 98 % (brut) CCM : 0,50 ; S.C.
RMN : 0,20-0,85 (m, 5H) ; 2,31 (m, 2H) ; 2,45 (s, 3H) ; 3,00 (d, 2H) ; 5,81 (dt, 1H) ; 6,35-6,51 (m, 3H) ; 7,28 (s, 5H) ; 7,48 (m,1H)

VI.8/trans-1-[2-cyano-2-(2-furyl)-N,N-diméthylaminoéthyl] -2-phényl-cyclopropane.

(R1 = 2-furyle ; R3 = R4 = CH3 ; R5 = C6H5 ; Q = cyclopropane 1,2 diyle)
A partir de XI.4 et de VIII.5
Rdt = 91 % (brut) CCM : 0,25-0,30 ; S.C.
RMN : 0,46-1,43 (m, 4H) ; 2,17-2,30 (m, 8H) ; 6,35 (m, 1H) ; 6,62 (m, 1H) ; 7,20 (m,5H) ; 7,45 (m, 1H)

VI.9/1-cyano-1-N,N-diméthylamino-1-(2-furyl)-4-(2-thiényl)-but-3-ène.

(R1 = 2-furyle ; R3 = R4 = CH3 ; R5 = 2-thiènyle ; Q = -CH=CH-)
A partir de XI.4 et de VIII.1
Rdt = 96 % (brut) CCM : 0,40-0,45 ; S.C.
RMN : 2,31 (s, 6H) ; 2,98 (d, 2H) ;5,68 (dt, 1H) ; 6,31-6,61 (m, 3H) ; 6,90-7,18 (m, 3H) ; 7,49 (m, 1H)

VI.10/1-cyano-1-N,N-diméthylamino-4-phényl-1-(2-thiényl) -but-3-ène.

(R1 = 2-thiènyle ; R3 = R4 = CH3 ; R5 = C6H5 ; Q = -CH=CH-)
A partir de XI.5 et de 3-phényl-1-chloropropène
Rdt = 91% (brut) CCM : 0,45-0,50 ; S.C.
RMN : 2,45 (s,6H) ; 2,88 (d, 2H) ;5,82 (dt, 1H) ; 6,43 (d, 1H) ; 6,88-7,01 (m, 1H) ; 7,28 (m, 7H)

VI.11/trans-1-[2-cyano-2-[2-thiényl)-N,N-diméthylamino-éthyl]-2-phényl-cyclopropane.

(R1 = 2-thiènyle ; R3 = R4 = CH3 ; R5 = C6H5 ; Q = cyclopropane 1,2 diyle)
A partir de XI.5 et de VIII.5
Rdt = 79 % (brut) CCM : 0,80-0,85 ; S.D.
RMN : 0,65-1,40 (m, 4H) ; 2,10-2,25 (m, 2H) ; 2,37 (s, 6H) ; 6,73-7,32 (m, 8H)

VI.12/1-cyano-1-N,N-diméthylamino-4-(2-furyl)-1-(2-thiényl)-but-3-ène.

(R1 = 2-thiènyle ; R3 = R4 = CH3 ; R5 = 2-furyle ; Q = -CH=CH-)
A partir de XI.5 et de VIII.3
Rdt = 79 % (brut) CCM : 0,50 ; S.C.
RMN : 2,38 (s, 6H) ; 2,90 (d, 2H) ; 5,78 (dt, 1H) ; 6,15-6,33 (m, 3H) ; 6,89-7,05 (m, 1H) ; 7,20-7,30 (m, 3H)

VI.13/1-cyano-1-N,N-diméthylamino-1,4-di(2-thiényl)-but-3-ène.

(R1 = R5 = 2-thiènyle ; R3 = R4 = CH3 ; Q = -CH=CH-)

A partir de XI.5 et de VIII.3
Rdt = 84 % (brut) CCM : 0,50 ; S.C.
RMN : 2,38 (s, 6H) ; 2,80 (d, 2H) ; 5,67 (dt, 1H) ; 6,51 (d, 1H) ; 6,88-7,38 (m, 6H)

VI.14/1-cyano-1-N,N-diméthylamino-1-(2-thiényl)-4-(3-thiényl)-but-3-ène.

(R1 = 2-thiènyle ; R3 = R4 = CH3 ; R5 = 3-thiènyle ; Q = -CH=CH-)
A partir de XI.5 et de VIII.2
Rdt = 87 % (brut) CCM : 0,50 ; S.C.
RMN : 2,35 (s, 6H) ; 2,9-3,2 (m, 2H) ; 5,7 (dt, 1H) ; 6,45 (d, 1H) ; 6,85-7,50 (m, 6H)

VI.15/1-cyano-1-N.N-diméthylamino-4-phényl-1-(3-thiényl) -but-3-ène.

(R1 = 3-thiènyle ; R3 = R4 = CH3 ; R5 = C6H5 ; Q = -CH=CH-)
A partir de XI.6 et de 3-phényl-1-chloropropène
Rdt = 87 % (brut) CCM : 0,40-0,45 ; S.C.
RMN : 2,35 (s,6H) ; 2,5-3,2 (m, 2H) ;5,5-6,0 (dt, 1H) ; 6,3 (d, 1H) ; 7,3 (s, 5H) ; 7,0-7,6 (m, 3H)

VI.16/trans-1-[2-cyano-2-(3-thiényl)-N,N-diméthylamino-ethyl] -2-phényl-cyclopropane.

(R1 = 3-thiènyle ; R3 = R4 = CH3 ; R5 = C6H5 ; Q = cyclopropane 1,2 diyle)
A partir de XI.6 et de VIII.4
Rdt = 83 % (brut) CCM : 0,45-0,50 ; S.B.
RMN : 0,50-2,30 (m, 6H) ; 2,27 (s, 6H) ; 6,7-7,5 (m, 8H)

VI.17/1-cyano-1-N,N-diméthylamino-1-(3-thiényl)-4-(2-thiényl)-but-3-ène.

(R1 = 3-thiènyle ; R3 = R4 = CH3 ; R5 = 2-thiènyle ; Q = -CH=CH-)
A partir de XI.6 et de VIII.1
Rdt = 85 % (brut) CCM : 0,50 ; S.C.
RMN : 2,35 (s, 6H) ; 2,9-3,2 (m, 2H) ; 5,7 (dt, 1H) ; 6,45 (d, 1H) ; 6,85-7,50 (m, 6H)

VI.18/1-cyano-1-N,N-diméthylamino-1,4-di(3-thiényl)-but-3-ène.

(R1 = 3-thiènyle ; R3 = R4 = CH3 ; R5 = 3-thiènyle ; Q = -CH=CH-)
A partir de XI.6 et de VIII.2
Rdt = 75 % (brut) CCM : 0,75 ; S.C.
RMN : 2,30 (s, 6H) ; 2,9 (m, 2H) ; 5,6 (dt, 1H) ; 6,40 (d, 1H) ; 7,0-7,45 (m, 6H)

VI.19/trans-1-(2-cyano-2-phényl-N,N-diméthylaminoéthyl) -2-(2-thiényl)-cyclopropane.

(R1 = C6H5 ; R3 = R4 = CH3 ; R5 = 2-thiènyle ; Q = cyclopropane 1,2 diyle)
A partir de XI.1 et de VIII.7
Rdt = 64 % (brut) CCM : 0,70 ; S.D.
RMN : 0,50-2,80 (m, 6H) ; 2,27 (s, 6H) ; 6,49-7,10 (m, 3H) ; 7,35 (m, 3H) ; 7,60 (m, 6H)

VI.20/trans-1-(2-cyano-2-(2-thiényl)-N,N-diméthylamino-éthyl]-2-(2-thiényl)-cyclopropane.

(R1 = R5 = 2-thiènyle ; R3 = R4 = CH3 ; Q = cyclopropane 1,2 diyle)
A partir de XI.5 et de VIII.7
Rdt = 58 % (brut) CCM : 0,50-0,55 : S.C.
RMN : 0,60-2,60 (m, 6H) ; 2,35 (s, 6H) ; 7,00 (m, 4H) ; 7,40 (m, 2H)

VI.21/trans-1-[2-cyano-2-(3-thiényl)-N,N-diméthylamino-éthyl]-2-(2-furyl)-cyclopropane.

(R1 = 3-thiènyle ; R3 = R4 = CH3 ; R5 = 2-furyl ; Q = cyclopropane 1,2 diyle)
A partir de XI.6 et de VIII.9
Rdt = 98 % (brut) CCM : 0,60 ; S.C.

VI.22/trans-1-[2-cyano-2-(2-furyl)-N,N-diméthylamino-éthyl]-2-(3-thiényl)-cyclopropane.

(R1 = 2-furyle ; R3 = R4 = CH3 ; R5 = 3-thiènyle ; Q = cyclopropane 1,2 diyle)
A partir de XI.4 et de VIII.8
Rdt = 95 % (brut) CCM : 0,45 ; S.C.
RMN : 0,40-2,40 (m,6H) ; 2,25 (s,6H) ; 6,20-6,60 (m, 6H)

ETHYLAMINES (I) DE L'INVENTION - EXEMPLES

- Mode opératoire général:

Dans un réacteur protégé de l'humidité et sous atmosphère d'azote, on introduit 2,26 l de solution 2,0 M de bromure d'éthylmagnésium (4,52 mol) dans le THF.

En 15 minutes, sous agitation et à la température ambiante voisine de 20°C, on introduit 275,0 g (1,0 mol) d'α-amino- acétonitrile (VI) en solution dans 1,6 l de THF.

Le mélange est agité à la même température durant 3 h puis on ajoute sans dépasser 20°C et avec précautions 4,5 l de solution aqueuse saturée en chlorure d'ammonium.

La phase aqueuse est décantée et extraite par 2 fois 650 ml de mélange hexanes-acétate d'éthyle 1-3 (v/v).

Les phases organiques réunies sont extraites par 2 fois 600 ml de solution HCl N. Les phases aqueuses acides réunies sont alcalinisées par une solution concentrée d'hydroxyde de sodium puis le mélange est extrait par 3 fois 650 ml de mélange hexanes-acétate d'éthyle. Les phases organiques réunies sont lavées à l'eau, séchées sur MgSO4 puis évaporées sous vide. Le produit résiduel est purifié par cristallisation ou par la préparation puis la purification d'un de ses sels d'addition qui, le plus fréquemment, est le chlorhydrate.

Ce mode expérimental est appliqué aux intermédiaires VI.1 à VI.22 avec le bromure d'éthylmagnésium pour obtenir les éthylamines (I) de l'invention décrites aux exemples 1 à 22.

Exemple 1 : trans 1-(2-N,N-diméthylamino-2-phényl-butyl) -2-phényl-cyclopropane

(R1 = R5 = C6H5 ; R2 = C2H5 ; R3 = R4 = CH3 ; Q = cyclopropane 1,2 diyle)
A partir du composé intermédiaire VI.1
Rdt = 47 % Eb/13 Pa = 142-152°C CCM : 0,60 ; S.
IR (film) : 3080, 3060, 3020, 2980, 2960, 2875, 2820, 2780, 1579, 1490, 1452, 1444, 1352, 1220, 1180, 1085, 1025, 995, 750, 690 cm-1
RMN : 0,65-2,15 (m, 11H) ; 2,26 (d, 6H) ; 6,87-7,50 (m, 10H)
Anal. (C21H27N) C, H, N

Exemple 2 : trans 1-[2-N,N-diméthylamino-2-(3,4,5-triméthoxyphényl)-butyl]-2-phényl-cyclopropane

(R1 = 3,4,5(CH30)3-C6H2 ; R2 = C2H5 ; R3 = R4 = CH3 ; R5 = C6H5 ; Q = cyclopropane 1,2 diyle)
A partir du composé intermédiaire VI.2
Rdt = 38 % CCM : 0,35 ; S.C
IR (film) : 3036, 2940, 2860, 2780, 1600, 1585, 1500, 1460, 1400, 1320, 1240, 1185, 1160, 1140, 1020, 750, 700 cm-1
RMN : 0,62-0,95 (m, 6H) ; 1,41-1,61 (m, 1H) ; 1,87-2,02 (m, 4H) ; 2,31 (d, 6H) ; 3,70-3,88(t, 9H) ; 6,66 (s,2H); 6,90-7,22 (m, 5H)
Anal. (C24H33NO3) C, H, N, O

Exemple 3 : 4-N,N-diméthylamino-4-phényl-1-(2-thiényl)-hex-1-ène

(R1 = C6H5 ; R2 = C2H5 ; R3 = R4 = CH3 ; R5 =2-thiènyle; Q = -CH=CH-)
A partir du composé intermédiaire VI.3
Rdt = 62 % F = 77-78°C CCM : 0,60-0,65 ; S.C
IR (KBr) : 3220, 2960-2880, 1465, 1380, 950, 760, 730, 700 cm-1
RMN : 0,72 (t, 3H) ; 1,90 (q, 2H) ; 2,25 (s, 6H) ; 2,80 (d, 2H) ; 5,90-6,22 (dt, 1H) ; 6,58 (d, 1H) ; 6,89-7,41 (m, 8H)
Anal. (C18H23NS) C, H, N, S

Exemple 4 : 4-N,N-diméthylamino-4-phényl-1-(3-thiényl)-hex-1-ene

(R1 = C6H5 ; R2 = C2H5 ; R3 = R4 = CH3 ; R5 =3-thiènyle; Q = -CH=CH-)

A partir du composé intermédiaire VI.4

Rdt = 58 % F = 95-96°C CCM : 0,50

IR (sol. CCl4) : 3080, 3040, 2980, 2930, 2850, 2820, 2775, 1500, 1470, 1460, 1450, 1250, 1190, 1140, 1100, 1040, 1020, 980 cm-1

RMN : 0,78 (t, 3H) ; 1,95 (q, 2H) ; 2,26 (s, 6H) ; 2,87 (d, 2H) ; 5,90-6,26 (dt, 1H) ; 6,55 (d, 1H) ; 7,05-7,50 (m, 8H)

Anal. (C18H23NS) C, H, N, S

Exemple 5 : trans 1-(2-N,N-diméthylamino-2-phényl-butyl)-2-(3,4,5-triméthoxyphényl)-cyclopropane

(R1 = C6H5 ; R2 = C2H5 ; R3 = R4 = CH3 ; R5 = 3,4,5 (CH30)3-C6H2 ; Q =cyclopropane 1,2 diyle)

A partir du composé intermédiaire VI.5

Rdt = 37 % CCM : 0,45 ; S.M.

IR (KBr) : 3060, 2980, 2930, 2780, 2740, 2400, 1585, 1515, 1470, 1420, 1320, 1255, 1235, 1150, 1130, 1010, 910, 850, 810, 760, 705 cm-1

RMN : 0,95-1,15 (m, 6H) ; 1,88 (m,2H) ; 2,48 (m, 1H) ; 2,71 (s, 6H) ; 3,30 (m, 2H) ; 3,77 (d, 9H) ; 6,33 (s,2H) 7,50-7,82 (m, 5H)

- Chlorhydrate : F = 205-208°C

Anal. (C241H33NO3.HCl) C, H, N, O, Cl

Exemple 6 : trans 1-[2-N,N-diméthylamino-2-(2-pyridyl) -butyl]-2-phényl-cyclopropane

(R1 = 2-pyridyle ; R2 = C2H5 ; R3 = R4 = CH3 ; R5 =C6H5; Q = cyclopropane 1,2 diyle)

A partir du composé intermédiaire VI.6

Rdt = 52 % Eb/13 Pa = 130°C CCM : 0,30 ; S.E.

IR (film) : 3060, 3020, 2970, 2940, 2875, 2820, 1610, 1590, 1570, 1500, 1465, 1430, 1150, 1100, 1050, 1000,915, 785, 740, 700 cm-1

RMN : 0,66-0,91 (m, 5H) ; 1,44-1,63 (m,2H) ; 1,80-2,24 (m, 4H) ; 2,30 (s, 6H) ; 6,89-7,35 (m, 6H) ; 7,52-7,69 (m, 2H) 8,62 (d, 1H)

Anal. (C20H26N2) C, H, N

Exemple 7 :4-N,N-diméthylamino-4-(2-furyl)-1-phényl-hex-1-ene

(R1 = 2-furyle ; R2 = C2H5 ; R3 = R4 = CH3 ; R5 = C6H5 ; Q = -CH=CH-)

A partir du composé intermédiaire VI.7

Rdt = 37 % CCM : 0,35 ; S.C.

IR (film) : 3020, 2980, 2940, 2880, 2820, 2780, 1600, 1575, 1495, 1470, 1450, 1160, 1040, 968, 800, 735, 690 cm-1

RMN : 1,81 (t, 3H) ; 1,86 (q,2H) ; 2,20 (s, 6H) ; 2,62-2,85 (m, 2H) ; 5,98-6,58 (m, 4H) ; 7,21-7,38 (m, 6H)

Anal. (C18H23NO) C, H, N, O

Exemple 8 : trans 1-[2-N,N-diméthylamino-2-(2-furyl) -butyl] -2-phényl-cyclopropane

(R1 = 2-furyle ; R2 = C2H5 ; R3 = R4 = CH3 ; R5 =C6H5; Q = cyclopropane 1,2 diyle)

A partir du composé intermédiaire VI.8

Rdt = 65 % CCM : 0,40 ; S.A

IR (film) : 3060, 3020, 2980, 2960, 2870, 2820, 2780, 1610, 1500, 1465, 1380, 1160, 1020, 940, 887, 805, 740, 700 cm-1

RMN : 0,80-0,97 (m, 6H) ; 1,23-1,70 (m, 1H) ; 1,91-2,03 (m, 4H) ; 2,16 (s, 6H) ; 6,06 (d, 1H) ; 6,26 (dd, 1H) ; 6,80-7,20 (m, 5H) ; 7,32 (d, 1H)

Anal. (C19H26NO) C, H, N, O

Exemple 9 : 4-N,N-diméthylamino-4-(2-furyl)-1-(2-thiényl)-hex-1-ène

(R1 = 2-furyle ; R2 = C2H5 ; R3 = R4 = CH3 ; R5 = 2-thiènyle ; Q = -CH=CH-)

A partir du composé intermédiaire VI.9
Rdt = 56 % F = 68-69°C CCM : 0,50-0,55 ; S.C
IR (nujol) : 2880-2940, 2840, 1460, 1370, 1150, 1040, 1020, 1000, 955, 850, 800, 740, 700, 695 cm-1
RMN : 0,85 (t, 3H) ; 1,90 (q, 2H) ; 2,25 (s, 6H) ; 2,80 (m, 2H) ; 5,80-6,12 (dt, 1H) ; 6,18 (m, 1H) ; 6,34 (m, 1H) ; 6,60 (d, 1H) ; 6,90-7,12 (m, 3H) ; 7,40 (d, 1H)
Anal. (C16H21NOS) C, H, N, O, S

Exemple 10 : 4-N,N-diméthylamino-1-phényl-4-(2-thiényl) -hex-1-ène

(R1 = 2-thiènyle ; R2 = C2H5 ; R3 = R4 = CH3 ; R5 =C6H5 ; Q = -CH=CH-)
A partir du composé intermédiaire VI.10
Rdt = 32 % CCM : 0,45-0,50 ; S.C
IR (film) : 3070, 3050, 3010, 2960, 2930, 2860, 2810, 2770, 1590, 1580, 1490, 1440, 1240, 960, 820, 735, 685 cm-1
RMN : 1,87 (t, 3H) ; 1,94 (q,2H) ; 2,24 (s, 6H) ; 2,86 (d, 2H) ; 6,05-6,63 (ddt, 2H) ; 6,87-7,07 (m, 2H) ; 7,17-7,37 (m, 6H)
Anal. (C18H23NS) C, H, N, S

Exemple 11 : trans 1-[2-N,N-diméthylamino-2-(2-thiényl) -butyl]-2-phényl-cyclopropane

(R1 = 2-thiènyle ; R2 = C2H5 ; R3 = R4 = CH3 ; R5 =C6H5 ; Q = cyclopropane 1,2 diyle)
A partir du composé intermédiaire VI.11
Rdt = 58 % Eb/0,1 = 155-167°C CCM : 0,60 ; S.C
IR (film) : 3060, 3020, 2970, 2930, 2870, 2820, 2780, 1610, 1500, 1460, 1375, 1250, 1240, 1225, 1090, 1040, 1030, 850, 830, 750, 700 cm-1
RMN : 0,75-0,98 (m, 6H) ; 1,55-1,77 (m, 1H) ; 2,00-2,10 (m, 4H) ; 2,23 (s, 6H) ; 6,83-7,26 (m, 8H)
Anal. (C19H26NS) C, H, N, S

Exemple 12 : 4-N,N-diméthylamino-1-(2-furyl)-4-(2-thiényl)-hex-1-ène

(R1 = 2-thiènyle ; R2 = C2H5 ; R3 = R4 = CH3 ; R5 = 2-furyle ; Q = -CH=CH-)
A partir du composé intermédiaire VI.12
Rdt = 51 % F = 59-60°C CCM : 0.50-0,55 ; S.C
IR (nujol) : 2940-2900, 2840, 1470, 1450, 1380, 1150, 1110, 1060, 740, 730, 700 cm-1
RMN : 0,85 (t, 3H) ; 1,95 (q, 2H) ; 2,25 (s, 6H) ; 2,85 (m, 2H) ; 6,10-6,35 (m, 4H) ; 6,85-7,06 (m, 2H) ; 7,23-7,32 (m, 2H)
Anal. (C16H21NOS) C, H, N, O, S

Example 13 : 4-N,N-diméthylamino-1,4-di(2-thiényl) -hex-1-ène

(R1 = 2-thiènyle ; R2 = C2H5 ; R3 = R4 = CH3 ; R5 = 2-thiènyle ; Q = -CH=CH-)
A partir du composé intermédiaire VI.13
Rdt = 65 % F = 69-71°C CCM : 0,60 ; S.C
IR (nujol) : 2820-2980, 1455, 1375, 1040, 995, 960, 850, 820, 720, 690 cm-1
RMN : 0,72 (t, 3H) ; 1,90 (q, 2H) ; 2,25 (s, 6H) ; 2,80 (d, 2H) ; 6,08 (dt, 1H) ; 6,60 (d, 1H) ; 6,82-7,25 (m, 6H) Anal. (C16H21NS2) C, H, N, S

Example 14 : 4-N,N-diméthy)amino-4-(2-thiényl)-1-(3-thiényl)-hex-1-ène

(R1 = 2-thiènyle ; R2 = C2H5 ; R3 = R4 = CH3 ; R5 = 3-thiènyle ; Q = -CH=CH-)
A partir du composé intermédiaire VI.14
Rdt = 61 % F = 85,5-86°C CCM : 0,40 ; S.M
IR (CCl4) : 2970, 2930, 2860, 2820, 2780, 1550, 1250, 1225, 1010, 960 cm-1
RMN : 0,90 (t, 3H) ; 1,95 (q, 2H) ; 2,25 (s, 6H) ; 2,85 (d, 2H) ; 5,90-6,25 (m, 1H) ; 6,60 (d, 1H) ; 6,90-7,35 (m, 6H)
Anal. (C16H21NS2) C, H, N, S

Exemple 15 : 4-N,N-diméthylamino-1-phényl-4-(3-thiényl) -hex-1-ène

(R1 = 3-thiènyle ; R2 = C2H5 ; R3 = R4 = CH3 ; R5 = C6H5 Q = -CH=CH-)
A partir du composé intermédiaire VI.15
Rdt = 45 % F = 91°C CCM : 0,60-0,65 ; S.E
IR (KBr) : 3100, 3020, 2950, 2930, 2850, 2810, 2770, 1590, 1490, 1470, 1450, 1290, 1245, 1000, 962, 843, 780, 690, 665 cm-1
RMN : 0,83 (t, 3H) ; 1,92 (q,2H) ; 2,20 (s, 6H) ; 2,75-3,00 (d, 2H) ; 6,00-6,65 (m, 2H) ; 6,90-7,45 (m, 8H)
Anal. (C18H23NS) C, H, N, S

Exemple 16 : trans 1-[2-N,N-diméthylamino-2-(3-thiényl) -butyl]-2-phényl-cyclopropane

(R1 = 3-thiènyle ; R2 = C2H5 ; R3 = R4 = CH3 ; R5 = C6H5; Q = cyclopropane 1,2 diyle)
A partir du composé intermédiaire VI.16
Rdt = 47 % Eb/6,6 Pa = 142°C CCM : 0,30 ; S.B
IR (film) : 3050, 3010, 2960, 2930, 2860, 2810, 2770, 1600 1492, 1455, 1087, 1028, 856, 838, 772, 745, 691, 668 cm-1
RMN : 0,85-1,05 (m, 6H) ; 1,55-2,10 (m, 5H) ; 2,25 (s, 6H) ; 6,90-7,50 (m, 8H)
Anal. (C19H26NS) C, H, N, S

Exemple 17 : 4-N,N-diméthylamino-1-(2-thiényl)-4-(3-thiényl) -hex-1-ène

(R1 = 3-thiènyle ; R2 = C2H5 ; R3 = R4 = CH3 ; R5 = 2-thiènyle ; Q = -CH=CH-)
A partir du composé intermédiaire VI.17
Rdt = 64 % F = 91-92°C CCM : 0,50 ; S.E
IR (CCl4) : 2960, 2920, 2810, 2775, 1545, 1245, 1000, 950 cm-1
RMN : 0,85 (t, 3H) ; 1,95 (q, 2H) ; 2,2 (s, 6H) ; 2,8 (d, 2H) ; 5,9-6,25 (m, 1H) ; 6,65 (d, 1H) ; 6,9-7,35 (m, 6H)
Anal. (C16H21NS2) C, H, N, S

Exemple 18 : 4-N,N-diméthylamino-1,4-di(3-thiényl) -hex-1-ène

(R1 = 3-thiènyle ; R2 = C2H5 ; R3 = R4 = CH3 ; R5 = 3-thiènyle ; Q = -CH=CH-)
A partir du composé intermédiaire VI.18
Rdt = 59 % F = 104-105°C CCM : 0,40 ; S.E
IR (CCl4) : 2970, 2930, 2870, 2820, 2780, 1550, 1255, 1225, 1020, 960, 775 cm-1
RMN : 0,85 (t, 3H) ; 1,95 (q, 2H) ; 2,2 (s, 6H) ; 2,85 (d, 2H) ; 5,9-6,6 (m, 2H) ; 7,05-7,40 (m, 6H)
Anal. (C16H21NS2) C, H, N, S

Exemple 19 : trans 1-(2-N,N-diméthylamino-2-phényl-butyl)-2-(2-thiényl)-cyclopropane

(R1 = C6H5 ; R2 = C2H5 ; R3 = R4 = CH3 ; R5 = 2-thiènyle Q = cyclopropane 1,2 diyle)
A partir du composé intermédiaire VI.19
Rdt = 66 % CCM : 0,25 ; S.D
IR (film) : 3060, 2980, 2960, 2820, 2780, 1450, 850, 760, 700 cm-1
RMN : 0,70-0,90 (m, 6H) ; 1,62-1,85 (m, 1H) ; 1,91-2,08 (m, 4H) ; 2,21 (s, 6H) ; 6,61 (t, 1H) ; 6,81 (m, 1H) ; 6,98 (d, 1H) ; 7,21-7,40 (m, 5H)
Anal. (C19H25NS) C, H, N, S

Exemple 20 : trans 1-[2-N,N-diméthylamino-2-(2-thiényl)-butyl)-2-(2-thiényl)-cyclopropane

(R1 = R5 = 2-thiènyle ; R2 = C2H5 ; R3 = R4 = CH3 ; Q = cyclopropane 1,2 diyle)
A partir du composé intermédiaire VI.20
Rdt = 45 % CCM : 0,30 ; S.D
IR (film) : 3060, 2960, 2920, 2870, 2860, 2810, 1660, 1530, 1450, 1235, 1040, 845, 820, 690 cm-1
RMN : 0,85-1,00 (m, 6H) ; 1,71-2,10 (m, 5H) ; 2,25 (s, 6H) ; 6,70-7,28 (m, 6H)
Anal. (C17H23NS2) C, H, N, S

Exemple 21 : trans 1-[2-N,N-diméthylamino-2-(3-thiényl)-butyl)-2-(2-furyl)-cyclopropane

(R1 = 3-thiènyle ; R2 = C2H5 ; R3 = R4 = CH3 ; R5 = 2-furyle; Q = cyclopropane 1,2 diyle)

A partir du composé intermédiaire VI.21

Rdt = 26 % CCM : 0,20 ; S.D

IR (film) : 3060, 2960, 2930, 2865, 2810, 2765, 1595, 1510, 1375, 1250, 1090 cm-1

RMN : 0,60-2,20 (m, 11H) ; 2,21 (s, 6H) ; 5,80-6,00 (m, 1H) ; 6,20-6,40 (m, 1H) ; 7,10-7,20 (m, 2H) ; 7,207,50 (m, 2H)

Anal. (C17H23NOS) C, H, N, O, S

Exemple 22 : trans 1-[2-N,N-diméthylamino-2-(2-furyl)-butyl]-2-(3-thiényl)-cyclopropane

(R1 = 2-furyle ; R2 = C2H5 ; R3 = R4 = CH3 ; R5 = 3-thiènyle; Q = cyclopropane 1,2 diyle)

A partir du composé intermédiaire VI.22

Rdt = 30 % CCM : 0,30 ; S.D

IR (film) : 3110, 3070, 2940, 2870, 2820, 2780, 1470, 1460, 1160, 1020, 775, 740 cm-1

RMN : 0,50-1,10 (m, 6H) ; 1,30-2,10 (m, 5H) ; 2,20 (s, 6H) ; 6,10-6,20 (m, 1H) ; 6,30-6,50 (m, 1H) ; 6,60-6,80 (m, 1H) ; 6,80-6,90 (m, 1H) ; 7,10-7,30 (m, 1H) ; 7,40-7,50 (m, 1H)

Anal. (C17H23NOS) C, H, N, O, S

Les essais toxicologiques et pharmacologiques réalisés avec les éthylamines de l'invention de formule (I) décrites dans les exemples 1 à 22 qui précèdent mettent en évidence leur faible toxicité de même que d'intéressantes propriétés psychotropes qui rendent ces composés utiles pour le traitement des affections neuropsychiques.

L'étude de la toxicité des produits de l'invention est recherchée chez la souris par voie orale par la détermination approchée de leur DL 50, qui est la dose léthale provoquant une mortalité de 50 % chez les animaux dans les conditions de l'expérience. Elle est réalisée sur des lots de quatre souris "Swiss" mâles d'un poids de 20 g environ mises à jeun la veille de l'essai.

Chaque détermination est effectuée avec quatre doses de produits correspondant respectivement à une administration de 100, 300, 600 et 1000 mg de produit, exprimé sous forme de base, par kg d'animal.

Il est ainsi constaté que les produits de l'invention ont en général une toxicité aigüe de DL50 supérieure ou égale à 1.000 mg/kg. Exceptionnellement, quelques composés présentent une DL50 d'environ 600mg/kg.

Les propriétés psychotropes des composés ont été determinées par la protection des convulsions induites par la picrotoxine chez la souris, réalisée selon une méthode dérivée de celle de Krall et coll., "Epilepsia", 1978, 19, p.409-428.

L'administration de picrotoxine provoque chez l'animal une crise convulsive caractérisée par un syndrome d'extension myoclonique suivi de l'extension des membres conduisant à la mort de l'animal. Certaines substances, notamment celles actives sur le complexe GABA/ benzodiazépines/Cl-ionophore permettent de protéger les animaux de cette crise convulsive.

Pratiquement l'étude est réalisée sur des lots de 10 souris "Swiss" mâles, d'un poids de 20 g environ auxquelles on administre le produit à étudier en solution aqueuse soit par voie intrapéritonéale (i.p.) sous un volume de 0,2 ml de solution par animal, soit par voie orale (p.o) sous un volume de 2,0 ml de solution par animal.

On pratique ensuite une injection par voie intrapéritonéale d'une solution de picrotoxine à raison de 24 mg/kg sous un volume de 0,2 ml par animal, soit 30 minutes après l'administration du produit par voie intrapéritonéale, soit 60 minutes après l'administration du produit par voie orale. La dose de produit injectée provoquant une crise clonique qui conduit à la mort des animaux non traités. Dans les conditions du test, on observe chez les animaux traités la suppression de la phase tonique d'extension. Les résultats sont exprimés :

– soit en pourcentage d'animaux protégés de cette phase sous l'action de 50 mg/kg du composé à l'étude administrée par voie i.p. ou 100 mg/kg par voie p.o.,

– soit en DE50 pour chacune de ces voies, qui est la dose efficace de composé à l'essai, exprimée en mg/kg protégeant 50 % des animaux de cette phase d'extension.

La valeur significative des résultats étant généralement indiqué de la façon suivante :

* Résultat significatif à p. < 0,05

** " " à p. < 0,01

*** " hautement significatif à p. < 0,001

Les résultats de l'étude sont reportés au tableau qui suit :

Tableau : Résultats - Inhibition des convulsions

| Exemple | i.p. : % prot. ou DE50 | p.o. : % prot. ou DE50 |
|---|---|---|
| 1 | 70 % ** | 90 % *** |
| 2 | 70 % ** | N.T. |
| 3 | 70 % ** | 70 % ** |
| 4 | 70 % ** | 80 % ** |
| 6 | 70 % ** | 80 % *** |
| 7 | 90 % *** | 100 % *** |
| 8 | 80 % *** | 60 % * |
| 9 | 50 % * | N.T. |
| 10 | N.T. | 80 % ** |
| 11 | 60 % * | 50 % * |
| 12 | 70 % * | 50 % * |
| 13 | 90 % *** | 50 % * |
| 15 | 70 % * | 100 % *** |
| 16 | 100 % *** | N.T. |
| 17 | 90 % *** | 80 % ** |
| 18 | 80 % *** | 100 % *** |
| 19 | DE 50 = 42 | DE 50 = 75 |
| 22 | DE 50 = 20 | 50 % * |

N.T. Non testé

Ces résultats mettent en évidence, pour les produits de l'invention étudiés, et indépendamment de la voie d'administration utilisée une activité dans le test de protection aux convulsions induites par la picrotoxine chez

la souris.

Ces propriétés pharmacologiques, associées à la faible toxicité des composés de l'invention permettent d'envisager leur utilité, sous forme de médicaments, aux traitements préventifs et curatifs d'affections neurologiques, notamment des convulsions et/ou psychiques en général et notamment des états dépressifs, états anxieux et/ou psychotiques.

Présentés sous les formes pharmaceutiques, de façon habituelle les doses unitaires utiles sont comprises entre 1 et 500 mg et plus particulièrement entre 5 et 200 mg de produit selon la nature et la gravité de l'affection a traiter. Les doses thérapeutiques journalières peuvent être réparties en plusieurs prises et sont comprises entre 5 et 2000 mg de produit par jour. De façon générale une posologie journalière de 50 à 500 mg de produit répartis en deux à quatre prises est satisfaisante.

L'administration des produits de l'invention aux patients à traiter est réalisée sous la forme de médicaments de nature adaptée à l'affection à soigner. Selon les cas les préparations médicamenteuses seront, comme exemples non limitatifs, des comprimés, dragées, capsules, poudres, solutions, suspensions, gels ou suppositoires. Ces formes pharmaceutiques sont préparées à partir des produits sous forme de base ou de leurs sels et selon des méthodes couramment pratiquées dans cette industrie.

Généralement dans les formes médicamenteuses de nature solide, le principe actif représente de 5 à 90 % en poids du total de la forme terminée alors que les excipients pharmaceutiquement appropriés représentent de 95 à 10 %. Pour les formes liquides, ou pouvant être considérées comme telles, la quantité de principe actif est comprise entre 0,1 et 10 % en poids de la forme terminée alors que les excipients pharmaceutiquement appropriés peuvent représenter de 99,9 à 90 % en poids de cette forme.

A titre d'illustration il est présenté la formulation et la préparation de comprimés.

– Formule

| | |
|---|---|
| Composé de l'exemple 10 | 10,0 à 50,0 mg |
| Polyvinylpyrrolidone | 20,0 mg |
| Carboxyméthylamidon | 8,0 mg |
| Stéarate de magnésium | 2,0 mg |
| Silice colloïdale | 0,4 mg |
| Lactose en quantité suffisante pour | 200,0 mg |

– Préparation

Le principe actif est mélangé au lactose puis granulé avec la polyvinylpyrrolidone en solution. Les grains sont séchés et tamisés sur une grille d'ouverture 1 mm. Le carboxyméthylamidon est mélangé à la silice colloïdale puis ajouté aux granulés. On mélange ensuite intimement avec le stéarate de magnésium puis comprime à raison de 200,0 mg par comprimé.

## Revendications

1. Ethylamines substituées de formule générale (I)

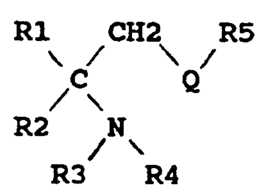

(I)

dans laquelle :

Q représente un groupe éthylène -1,2-diyle (-CH=CH-) ou un groupe cyclopropane-1,2-diyle

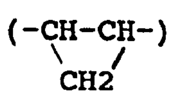

,

R1 est un hétérocycle aromatique de 5 à 7 chaînons dans lequel l'unique hétéroatome est l'azote, l'oxygène ou le soufre,
ou est un radical phényle éventuellement mono, di ou tri-substitué par des radicaux alkyle ou alkoxy inférieurs identiques ou différents, sous réserve que Q soit un groupe cyclopropane-1,2-diyle ou sous réserve que R5 représente un radical hétérocyclique aromatique tel qu'il est défini ci-dessous,
R2 est alkyle inférieur,
R3 et R4, identiques ou différents, sont l'hydrogène ou des radicaux alkyle inférieur sans toutefois représenter tous deux l'hydrogène,
R5 est un hétérocycle aromatique de 5 à 7 chaînons dans lequel l'unique hétéroatome est l'azote, l'oxygène ou le soufre,
ou est un radical phényle éventuellement mono, di ou tri-substitué par des radicaux alkyle ou alkoxy inférieurs identiques ou différents, et leurs sels d'addition avec des acides.

2. Ethylamines selon la revendication 1, caractérisées en ce que R1 est un radical thiényle.

3. Ethylamines selon la revendication 1, caractérisées en ce que R1 est un radical furyle.

4. Ethylamines selon l'une des revendications 1 à 3, caractérisées en ce que R5 est un radical phényle.

5. Ethylamines selon l'une des revendications 1 à 4, caractérisées en ce que R2 est un radical éthyle.

6. Ethylamines selon l'une quelconque des revendications 1 à 5, caractérisées en ce que R3 et R4 sont chacun un radical méthyle.

7. Procédé de préparation des éthylamines telles que définies à l'une des revendications 1 à 6, caractérisé en qu'il consiste,
- pour préparer une éthylamine de formule (I) dans laquelle R3 est un radical méthyle et R4 est l'hydrogène,
i) à réduire un isocyanate de formule (II) ou un isonitrile de formule (II') :

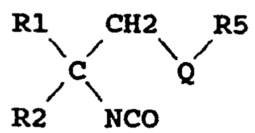

(II)

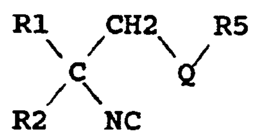

(II')

par un hydrure métallique ou organométallique (Hm.1) de formule générale (Hm) :

M1(t) M2 H(r) Rx(s) (Hm)

dans laquelle :

M1 représente un métal alcalin, de préférence le lithium ou le sodium et dont l'indice représentatif (t) a pour valeur 0 ou 1,

M2 est un métal du groupe III de la classification périodique des éléments, de préférence le bore ou l'aluminium,

(r) est l'indice représentatif du nombre d'atomes d'hydrogène de l'hydrure et a pour valeurs 1, 2, 3 ou 4,

Rx représente un groupe carbonitrile, un radical alkyle ou alkoxy inférieur dont l'indice représentatif (s) vaut 0, 1, 2 ou 3,

les indices : (t), (r) et (s) vérifiant la relation : (r) + (s) - (t) = 3,

ou bien à réduire un formamide de formule

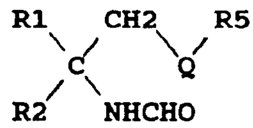

par un hydrure métallique (Hm.2) de formule (Hm) dans laquelle M2 est l'aluminium ou le bore lorsque (r) vaut 3 et (t) vaut 0,

- pour préparer une éthylamine de formule (I) dans laquelle R3 est un radical alkyle inférieur différent du méthyle et R4 est l'hydrogène,

i) à alkyler une amine de formule (III) :

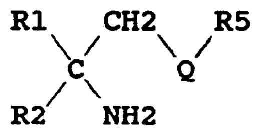 (III)

par une halogénure d'alkyle R3Z1, Z1 étant un radical chlore, brome ou iode, ou bien

ii) à réduire un carboxamide de formule (IV)

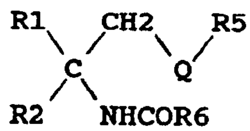 (IV)

dans laquelle le radical R6 est l'homologue carboné inférieur de R3 (R3 = -CH2-R6), par un hydrure métallique (Hm.2) précédemment défini,

- pour préparer une éthylamine de formule (I) dans laquelle R3 et R4 sont chacun un radical méthyle, i) à diméthyler une amine de formule (III) ci-dessus par le formaldéhyde et en présence soit d'acide formique soit d'un hydrure réducteur métallique ou organométallique (Hm.3) de formule (Hm) dans laquelle M2 est le bore, et de façon préférée Rx représente un groupe carbonitrile,

- pour préparer une éthylamine de formule (I) dans laquelle R3 et R4 sont chacun alkyle inférieur et sont différents,

i) à réduire par un hydrure métallique (Hm.2) précédemment défini un carboxamide de formule (V)

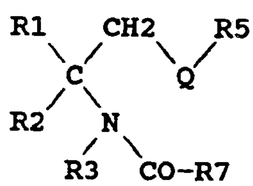 (V)

dans laquelle R7 est l'homologue carboné inférieur à R4 (R4 = -CH2-R7), ou bien

ii) à faire réagir un réactif organomagnésien de formule R2MgZ3 dans laquelle Z3 est un atome de chlore, de brome, d'iode avec un aminonitrile de formule (VI)

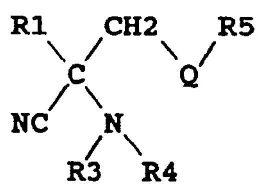 (VI)

- et, pour préparer une éthylamine de formule (I) dans laquelle R3 est alkyle inférieur et R4 est méthyle, i) à N-méthyler une éthylamine (I) dans laquelle R3 est alkyle inférieur et R4 est l'hydrogène par le formaldéhyde et un réducteur tel que l'hydrure métallique ou organométallique (Hm.3) précédemment défini

8. Médicament, caractérisé en ce qu'il comprend une éthylamine de formule générale (I) telle que définie dans l'une des revendications 1 à 6.

9. Utilisation d'une éthylamine de formule (I) pour l'obtention d'un médicament destiné à traiter les affections neurologiques et/ou psychiques.

10. Composés intermédiaires de formule (XX)

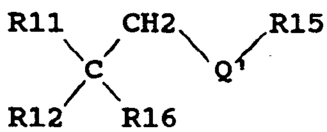

(XX)

dans laquelle :

Q' représente un groupe éthylène -1,2-diyle (-CH=CH-) ou un groupe cyclopropane-1,2-diyle

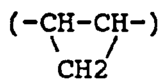

R11 est un hétérocycle aromatique de 5 à 7 chaînons dans lequel l'unique hétéroatome est l'azote, l'oxygène ou le soufre,
ou est un radical phényle éventuellement mono, di ou tri-substitué par des radicaux alkyle ou alkoxy inférieurs identiques ou différents, sous réserve que Q' soit un groupe cyclopropane-1,2-diyle ou sous réserve que R15 représente un radical hétérocyclique aromatique tel qu'il est défini ci-dessous,
R12 est alkyle inférieur ou un radical carbonitrile,
R15 est un hétérocycle aromatique de 5 à 7 chaînons dans lequel l'unique hétéroatome est l'azote, l'oxygène ou le soufre,
ou est un radical phényle éventuellement mono, di ou tri-substitué par des radicaux alkyle ou alkoxy inférieurs identiques ou différents,
R16 est un radical isocyanate -NCO ou un radical isonitrile -NC, ou encore un radical -N(R13)R14, dans lequel R13 et R14 identiques ou différents sont l'hydrogène, ou des radicaux alkyle inférieur ou encore des radicaux R6-CO- ou R7-CO- dans lesquels R6 et R7 représentent l'hydrogène ou un radical alkyle homologue inférieur carboné de R13 ou de R14, avec R13 = -CH2-R6 et R14 = -CH2-R7.

**Revendications pour l'Etat Contractant suivant: ES**

1. Procédé de préparation d'éthylamines substituées de formule générale (I)

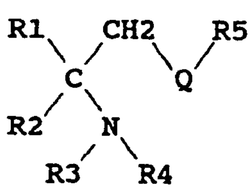

(I)

dans laquelle :

Q représente un groupe éthylène -1,2-diyle (-CH=CH-) ou un groupe cyclopropane-1,2-diyle

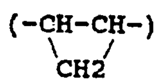 ,

R1 est un hétérocycle aromatique de 5 à 7 chaînons dans lequel l'unique hétéroatome est l'azote, l'oxygène ou le soufre,
ou est un radical phényle éventuellement mono, di ou tri-substitué par des radicaux alkyle ou alkoxy inférieurs identiques ou différents, sous réserve que Q soit un groupe cyclopropane-1,2-diyle ou sous réserve que R5 représente un radical hétérocyclique aromatique tel qu'il est défini ci-dessous,
R2 est alkyle inférieur,
R3 et R4, identiques ou différents, sont l'hydrogène ou des radicaux alkyle inférieur sans toutefois représenter tous deux l'hydrogène,
R5 est un hétérocycle aromatique de 5 à 7 chaînons dans lequel l'unique hétéroatome est l'azote, l'oxygène ou le soufre,
ou est un radical phényle éventuellement mono, di ou tri-substitué par des radicaux alkyle ou alkoxy inférieurs identiques ou différents, et leurs sels d'addition avec des acides, caractérisé en ce qu'il consiste,

- pour préparer une éthylamine de formule (I) dans laquelle R3 est un radical méthyle et R4 est l'hydrogène,
  i) à réduire un isocyanate de formule (II) ou un isonitrile de formule (II') :

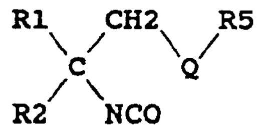
(II)

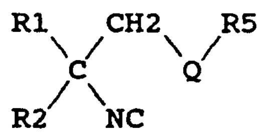
(II')

par un hydrure métallique ou organométallique (Hm.1) de formule générale (Hm) :

M1(t) M2 H(r) Rx(s) (Hm)

dans laquelle :

M1 représente un métal alcalin, de préférence le lithium ou le sodium et dont l'indice représentatif (t) a pour valeur 0 ou 1,

M2 est un métal du groupe III de la classification périodique des éléments, de préférence le bore ou l'aluminium,

(r) est l'indice représentatif du nombre d'atomes d'hydrogène de l'hydrure et a pour valeurs 1, 2, 3 ou 4,

Rx représente un groupe carbonitrile, un radical alkyle ou alkoxy inférieur dont l'indice représentatif (s) vaut 0, 1, 2 ou 3,

les indices : (t), (r) et (s) vérifiant la relation : (r) + (s) - (t) = 3

ou bien à réduire un formamide de formule

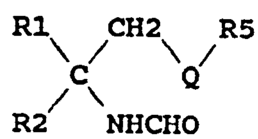

par un hydrure métallique (Hm.2) de formule (Hm) dans laquelle M2 est l'aluminium ou le bore lorsque (r) vaut 3 et (t) vaut 0,

- pour préparer une éthylamine de formule (I) dans laquelle R3 est un radical alkyle inférieur différent du méthyle et R4 est l'hydrogène,
  i) à alkyler une amine de formule (III) :

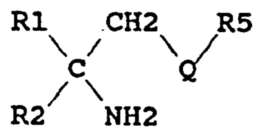

(III)

par une halogénure d'alkyle R321, 21 étant un radical chlore, brome ou iode, ou bien

ii) à réduire un carboxamide de formule (IV)

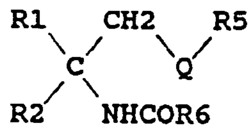

(IV)

dans lequel le radical R6 est l'homologue carboné inférieur de R3 (R3 = -CH2-R6), par un hydrure métallique (Hm.2) précédemment défini,

- pour préparer une éthylamine de formule (I) dans laquelle R3 et R4 sont chacun un radical méthyle, i) à diméthyler une amine de formule (III) ci-dessus par le formaldéhyde et en présence soit d'acide formique soit d'un hydrure réducteur métallique ou organométallique (Hm.3) de formule (Hm) dans laquelle M2 est le bore, et de façon préférée Rx représente un groupe carbonitrile,

- pour préparer une éthylamine de formule (I) dans laquelle R3 et R4 sont chacun alkyle inférieur et sont différents,

i) à réduire par un hydrure métallique (Hm.2) précédemment défini un carboxamide de formule (V)

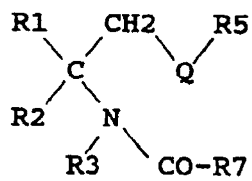

(V)

dans laquelle R7 est l'homologue carboné inférieur à R4 (R4 = -CH2-R7), ou bien

ii) à faire réagir un réactif organomagnésien de formule R2MgZ3 dans laquelle Z3 est un atome de chlore, de brome, d'iode avec un aminonitrile de formule (VI)

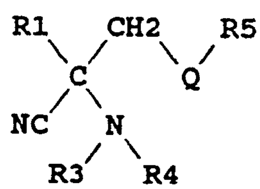

(VI)

- et, pour préparer une éthylamine de formule (I) dans laquelle R3 est alkyle inférieur et R4 est méthyle, i) à N-méthyler une éthylamine (I) dans laquelle R3 est alkyle inférieur et R4 est l'hydrogène par le formaldéhyde et un réducteur tel que l'hydrure métallique ou organométallique (Hm.3) précédemment défini.

2. Procédé suivant la revendication 1, caractérisé en ce que R1 est un radical thiényle.

3. Procédé suivant la revendication 1, caractérisé en ce que R1 est un radical furyle.

4. Procédé suivant l'une quelconque des revendications 1 à 3, caractérisé en ce que R5 est un radical phényle.

5. Procédé suivant l'une quelconque des revendications 1 à 4, caractérisé en ce que R2 est un radical éthyle.

6. Procédé suivant l'une quelconque des revendications 1 à 5, caractérisé en ce que R3 et R4 sont chacun un radical méthyle.

7. Procédé de préparation d'un médicament, caractérisé en ce qu'il consiste à mélanger une éthylamine de formule (I) telle que définie aux revendications 1 à 6 à un excipient pharmaceutiquement acceptable.

8. Utilisation d'une éthylamine de formule (I) pour l'obtention d'un médicament destiné à traiter les affections neurologiques et/ou psychiques.

9. Procédé de préparation de composés intermédiaires de formule (XX) :

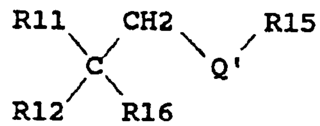

(XX)

dans laquelle :

Q' représente un groupe éthylène -1,2-diyle (-CH=CH-) ou un groupe cyclopropane-1,2-diyle

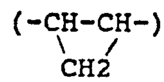

R11 est un hétérocycle aromatiqe de 5 à 7 chaînons dans lequel l'unique hétéroatome est l'azote, l'oxygène ou le soufre,
ou est un radical phényle éventuellement mono, di ou tri-substitué par des radicaux alkyle ou alkoxy inférieurs identiques ou différents, sous réserve que Q' soit un groupe cyclopropane-1,2-diyle ou sous réserve que R15 représente un radical hétérocyclique aromatique tel qu'il est défini ci-dessous,
R12 est alkyle inférieur ou un radical carbonitrile,
R15 est un hétérocycle aromatique de 5 à 7 chaînons dans lequel l'unique hétéroatome est l'azote, l'oxygène ou le soufre,
ou est un radical phényle éventuellement mono, di ou tri-substitué par des radicaux alkyle ou alkoxy inférieurs identiques ou différents,
R16 est un radical isocyanate -NCO ou un radical isonitrile -NC, ou encore un radical -N(R13)R14, dans laquelle R13 et R14 identiques ou différents sont l'hydrogène, ou des radicaux alkyle inférieur ou encore des radicaux R6-CO- ou R7-CO- dans lesquels R6 et R7 représentent l'hydrogène ou un radical alkyle homologue inférieur carboné de R13 ou de R14,
avec R13 = -CH2-R6 et R14 = -CH2-R7,
caractérisé en ce qu'il consiste,

- Pour préparer les composés (II) de formule (XX)

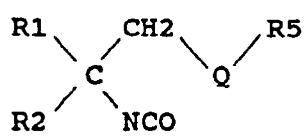

(II)

dans laquelle R1, R2, Q et R5 représentent les radicaux ou groupes ci-dessus,
à soumettre le composé (IX) de formule :

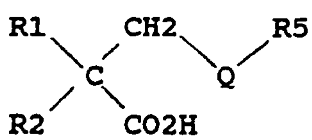

(IX)

à une réaction de Curtius,

- Pour préparer les composés (II') de formule :

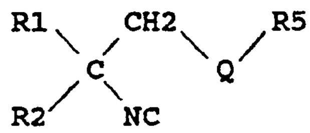

(II')

à soumettre le composé isonitrile :

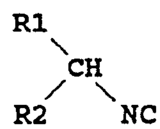

à une alkylation par un réactif (VIII) :

Z7-CH2-Q-R5

Z7 étant un halogène ou un radical alkylsulfonyloxy, Q et R5 ayant la même signification que ci-dessus,

- Pour préparer les éthylamines (III) de formule :

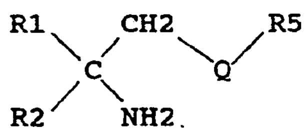

(III)

à hydrolyser soit un isocyanate de formule (II) ou un isonitrile de formule (II'),

- Pour préparer un carboxamide intermédiaire (IV) de formule :

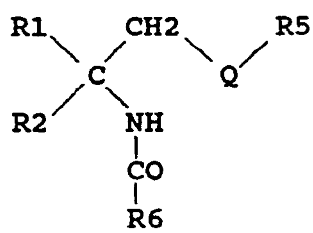

(IV)

à acyler une amine de formule (III) par un réactif (R6-CO)nZ2 dans lequel R6 est l'homologue carboné directement inférieur à R3 (R3 = -CH2-R6) et n a pour valeur 1 lorsque Z2 est un halogène comme le chlore ou le brome, ou est un radical hydroxyle, et n a pour valeur 2 lorsque Z2 représente un atome d'oxygène, et

-pour préparer un carboxamide intermédiaire (V) de formule :

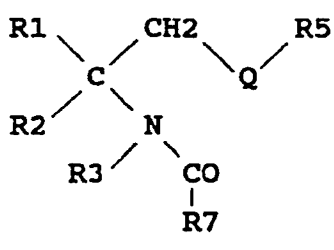

(V)

à acyler une amine (I) de formule :

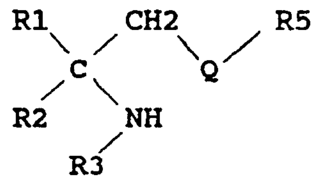

par un halogénure de formule R7-COZ5, dans laquelle R7 est alkyle inférieur et Z5 est le brome ou le chlore, et pour préparer un aminonitrile (VI) de formule :

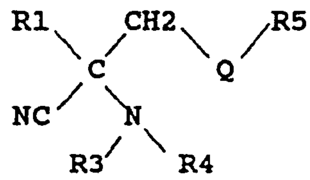

(VI)

à alkyler un aminonitrile (XI) de formule :

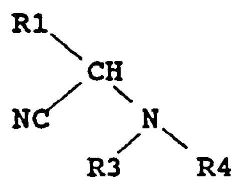

(XI)

par le réactif (VIII) déjà défini ci-dessus.

**Revendications pour l'Etat Contractant suivant: GR**

1. Ethylamines substituées de formule générale (I)

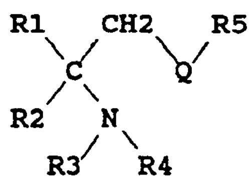

(I)

dans laquelle :

Q représente un groupe éthylène -1,2-diyle (-CH=CH-) ou un groupe cyclopropane-1,2-diyle

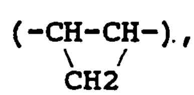

R1 est un hétérocycle aromatique de 5 à 7 chaînons dans lequel l'unique hétéroatome est l'azote, l'oxygène ou le soufre,
ou est un radical phényle éventuellement mono, di ou tri-substitué par des radicaux alkyle ou alkoxy inférieurs identiques ou différents, sous réserve que Q soit un groupe cyclopropane-1,2-diyle ou sous réserve que R5 représente un radical hétérocyclique aromatique tel qu'il est défini ci-dessous,
R2 est alkyle inférieur,
R3 et R4, identiques ou différents, sont l'hydrogène ou des radicaux alkyle inférieur sans toutefois représenter tous deux l'hydrogène,
R5 est un hétérocycle aromatique de 5 à 7 chaînons dans lequel l'unique hétéroatome est l'azote, l'oxygène ou le soufre,
ou est un radical phényle éventuellement mono di ou tri-substitué par des radicaux alkyle ou alkoxy inférieurs identiques ou différents, et leurs sels d'addition avec des acides.

2. Ethylamines selon la revendication 1, caractérisées en ce que R1 est un radical thiényle.

3. Ethylamines selon la revendication 1, caractérisées en ce que R1 est un radical furyle.

4. Ethylamines selon l'une quelconque des revendications 1 à 3, caractérisées en ce que R5 est un radical phényle.

5. Ethylamines selon l'une des revendications 1 à 4, caractérisées en ce que R2 est un radical éthyle.

6. Ethylamines selon l'une quelconque des revendications 1 à 5, caractérisées en ce que R3 et R4 sont chacun un radical méthyle.

7. Procédé de préparation des éthylamines telles que définies à l'une des revendications 1 à 6, caractérisées en qu'il consiste,
- pour préparer une éthylamine de formule (I) dans laquelle R3 est un radical méthyle et R4 est l'hydrogène,
i) à réduire un isocyanate de formule (II) ou un isonitrile de formule (II') :

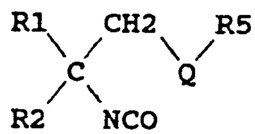

(II)

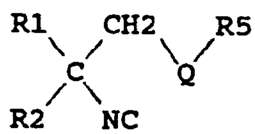

(II')

par un hydrure métallique ou organométallique (Hm.1) de formule générale (Hm) :

M1(t) M2 H(r) Rx(s) (Hm)

dans laquelle :

M1 représente un métal alcalin, de préférence le lithium ou le sodium et dont l'indice représentatif (t) a pour valeur 0 ou 1,

M2 est un métal du groupe III de la classification périodique des éléments, de préférence le bore ou l'aluminium,

(r) est l'indice représentatif du nombre d'atomes d'hydrogène de l'hydrure et a pour valeurs 1, 2, 3 ou 4,

Rx représente un groupe carbonitrile, un radical alkyle ou alkoxy inférieur dont l'indice représentatif (s) vaut 0, 1, 2 ou 3,

les indices : (t), (r) et (s) vérifiant la relation : (r) + (s) - (t) = 3,

ou bien à réduire un formamide de formule

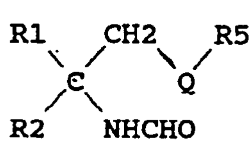

par un hydrure métallique (Hm.2) de formule (Hm) dans laquelle M2 est l'aluminium ou le bore lorsque (r) vaut 3 et (t) vaut 0,
- pour préparer une éthylamine de formule (I) dans laquelle R3 est un radical alkyle inférieur différent du

méthyle et R4 est l'hydrogène,

i) à alkyler une amine de formule (III) :

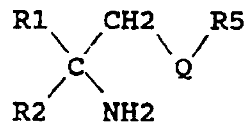

(III)

par un halogénure d'alkyle R3Z1, Z1 étant un radical chlore, brome ou iode, ou bien

ii) à réduire un carboxamide de formule (IV)

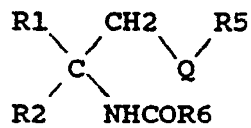

(IV)

dans laquelle le radical R6 est l'homologue carboné inférieur de R3 (R3 = -CH2-R6), par un hydrure métallique (Hm.2) précédemment défini,

- pour préparer une éthylamine de formule (I) dans laquelle R3 et R4 sont chacun un radical méthyle, i) à diméthyler une amine de formule (III) ci-dessus par le formaldéhyde et en présence soit d'acide formique soit d'un hydrure réducteur métallique ou organométallique (Hm.3) de formule (Hm) dans laquelle M2 est le bore, et de façon préférée Rx représente un groupe carbonitrile,

- pour préparer une éthylamine de formule (I) dans laquelle R3 et R4 sont chacun alkyle inférieur et sont différents,

i) à réduire par un hydrure métallique (Hm.2) précédemment défini un carboxamide de formule (V)

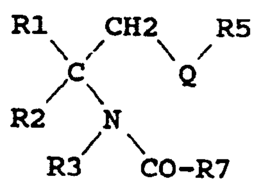

(V)

dans laquelle R7 est l'homologue carboné inférieur à R4 (R4 = -CH2-R7), ou bien

ii) à faire réagir un réactif organomagnésien de formule R2MgZ3 dans laquelle Z3 est un atome de chlore, de brome, d'iode avec un aminonitrile de formule (VI)

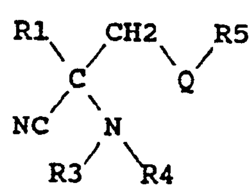

(VI)

- et, pour préparer une éthylamine de formule (I) dans laquelle R3 est alkyle inférieur et R4 est méthyle, i) à N-méthyler une éthylamine (I) dans laquelle R3 est alkyle inférieur et R4 est l'hydrogène par le formaldéhyde et un réducteur tel que l'hydrure métallique ou organométallique (Hm.3) précédemment défini.

**8.** Procédé de préparation d'un médicament, caractérisé en ce qu'il consiste à mélanger une éthylamine de formule (I) telle que définie aux revendications 1 à 6 à un excipient pharmaceutiquement acceptable.

**9.** Utilisation d'une éthylamine de formule (I) pour l'obtention d'un médicament destiné à traiter les affections neurologiques et/ou psychiques.

**10.** Composés intermédiaires de formule (XX)

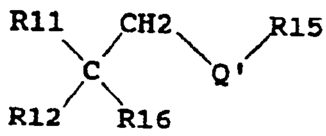

(XX)

dans laquelle :

Q' représente un groupe éthylène -1,2-diyle (-CH=CH-) ou un groupe cyclopropane-1,2-diyle

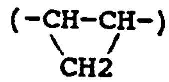

R11 est un hétérocycle aromatique de 5 à 7 chainons dans lequel l'unique hétéroatome est l'azote, l'oxygène ou le soufre,

ou est un radical phényle éventuellement mono, di ou tri-substitué par des radicaux alkyle ou alkoxy inférieurs identiques ou différents, sous réserve que Q' soit un groupe cyclopropane-1,2-diyle ou sous réserve que R15 représente un radical hétérocyclique aromatique tel qu'il est défini ci-dessous,

R12 est alkyle inférieur ou un radical carbonitrile,

R15 est un hétérocycle aromatique de 5 à 7 chainons dans lequel l'unique hétéroatome est l'azote, l'oxygène ou le soufre,

ou est un radical phényle éventuellement mono, di ou tri-substitué par des radicaux alkyle ou alkoxy inférieurs identiques ou différents,

R16 est un radical isocyanate -NCO ou un radical isonitrile -NC, ou encore un radical -N(R13)R14, dans lequel R13 et R14 identiques ou différents sont l'hydrogène, ou des radicaux alkyle inférieur ou encore des radicaux R6-CO- ou R7-CO- dans lesquels R6 et R7 représentent l'hydrogène ou un radical alkyle homologue inférieur carboné de R13 ou de R14, avec R13 = -CH2-R6 et R14 = -CH2-R7.

Office européen des brevets

# RAPPORT DE RECHERCHE EUROPEENNE

Numero de la demande

EP 91 40 0496

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 297 782 (TANABE)<br>* Revendications *<br>--- | 1-10 | C 07 D 333/20<br>C 07 D 307/52<br>C 07 D 307/54<br>C 07 D 333/24<br>C 07 D 213/38<br>C 07 D 213/57<br>C 07 C 211/27<br>C 07 C 217/74<br>C 07 C 255/43<br>C 07 D 409/06<br>A 61 K 31/38<br>A 61 K 31/34<br>A 61 K 31/395<br>A 61 K 31/13 |
| D,Y | EP-A-0 298 703 (TANABE)<br>* Revendications *<br>----- | 1-10 | |
| | | | **DOMAINES TECHNIQUES RECHERCHES (Int. Cl.5)** |
| | | | C 07 D 333/00<br>C 07 D 307/00<br>C 07 D 213/00<br>C 07 D 409/00<br>C 07 C 211/00<br>C 07 C 217/00<br>C 07 C 255/00<br>A 61 K 31/00 |

Le présent rapport a été établi pour toutes les revendications

| Lieu de la recherche | Date d'achèvement de la recherche | Examinateur |
|---|---|---|
| LA HAYE | 22-04-1991 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)